# EUROPÄISCHE PATENTSCHRIFT

(11) **EP 2 243 469 B1**
(45) Veröffentlichungstag und Bekanntmachung des Hinweises auf die Patenterteilung: **27.06.2012**
(21) Anmeldenummer: 10160424.7
(22) Anmeldetag: 20.04.2010
(51) Int. Cl.: A61K 9/00, A61K 9/20, A23L 1/00, A61K 8/67, A61K 36/00

(54) **Gefriergetrockneter Formkörper enthaltend Magnesiumascorbylphosphat**
Freeze-dried form body containing magnesium ascorbyl phosphate
Corps de formage séché par congélation comprenant du phosphate d'ascorbyle de magnésium

(30) Priorität: 22.04.2009 EP 09158475
(43) Veröffentlichungstag der Anmeldung: 27.10.2010
(73) Patentinhaber: Dr. Suwelack Skin & Health Care AG, 48727 Billerbeck (DE)
(72) Erfinder: Malessa, Dr. Ralf, 45134, Essen (DE)
(74) Vertreter: Gille Hrabal

(56) Entgegenhaltungen:
- WO-A-2008/020066
- WO-A-2009/014347
- FR-A- 2 886 845

## Beschreibung

Die Erfindung betrifft gefriergetrocknete Formkörper enthaltend Magnesiumascorbylphosphat und Alginat sowie gegebenenfalls einen oder mehrere Wirkstoffe und/oder Hilfsstoffe. Weiterhin betrifft die Erfindung Verfahren zur Herstellung dieser gefriergetrockneten Formkörper, die Kombination solcher gefriergetrockneten Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung.

Bei der pharmazeutischen und kosmetischen Anwendung und Verabreichung von Wirk- und Pflegestoffen oder anderen aktiven Substanzen spielt insbesondere die Wahl und Bereitstellung der geeigneten Darreichungsform zur Applikation der Wirk- und/oder Pflegestoffe eine zentrale Rolle. Dabei sind Faktoren zu beachten wie z. B. reproduzierbare und wirksame Wirkstoffgehalte, hohe Sicherheit bei der Lagerung und Applikation, gute und reproduzierbare Verfügbarmachung der Wirkstoffe nach der Applikation, gegebenenfalls kann eine Stabilisierung der enthaltenen Wirkstoffe gegen Abbau oder Zersetzung von Bedeutung sein, sowie aber auch die Bereitstellung der Wirkstoffe in einer optimal auf den jeweiligen Anwendungszweck abgestimmten Darreichungsform. Die Wahl der geeigneten Darreichungsform hängt dabei insbesondere auch ab von der Art und dem Ort der Applikation, der Zielgruppe der Endanwender und deren Eigenheiten, der Art und Höhe der Dosierung der Wirkstoffe oder beispielsweise auch den physikalischen und biochemischen Charakteristika der Wirkstoffe insbesondere in Hinblick auf ihre biologische Verfügbarkeit und systemische Wirkungsweise.

Einen besonders wichtigen Aspekt bei der Verabreichung von Wirk- und Aktivsubstanzen oder kosmetischen Pflegestoffen stellt dabei die Stabilisierung und Haltbarmachung der Zubereitungen dar. Besonders haltbare und stabile Darreichungsformen sind wasserfreie bzw. feste und trockene Zubereitungen, die außerdem besonders gute Lager- und Transporteigenschaften aufweisen. Gerade bei Verabreichungssystemen in getrockneter oder trockener Form tritt als zentrale Frage bei der Verabreichung am und im menschlichen oder tierischen Körper die Auflösung bzw. Löslichkeit der Darreichungsform und damit der Freisetzung und Verfügbarmachung der darin enthaltenen Wirk-, Pflege- oder Aktivstoffe auf. Die Anforderungen an die Art, Geschwindigkeit und den Ort der Auflösung variieren mit der speziellen Anwendung, den zu applizierenden Substanzen sowie gegebenenfalls weiteren zu berücksichtigenden Faktoren.

Insbesondere bei der kosmetischen Applikation von Wirkstoffen werden insbesondere Darreichungsformen gewünscht, die die äußerliche Verabreichung von Wirk-, Aktiv- und Pflegestoffen auf der Haut und dem Haar optimal ermöglicht, aber auch bei der pharmazeutischen Applikation von Wirkstoffen spielt die äußere Anwendung neben der oralen Verabreichung von Wirkstoffen eine große Rolle.

Zur schnellen und gezielten Applikation von Wirkstoffen, insbesondere von solchen, die eine gewisse Instabilität gegen äußere Faktoren wie Feuchtigkeit, Oxidation oder Temperatur aufweisen, sind wasserfreie Darreichungsformen ein probates Mittel. Von besonderem Interesse sind hierbei trockene Zubereitungen, die über ein besonders schnelles und vollständiges Auflöseverhalten verfügen. Dies ist besonders relevant bei der Verfügbarmachung von feuchtigkeitsinstabilen Wirkstoffen sowie generell bei der oralen Applikation von Wirkstoffen, die z. B. über die Mundschleimhaut aufgenommen werden sollen oder für Anwender, die mit dem Schlucken von Tabletten oder Kapseln Probleme haben.

Weitere Nachteile von bekannten trockenen Darreichungsformen wie Pulvergemischen, Granulaten, Tabletten oder Kapseln sind insbesondere die häufig schlechte bzw. langsame Löslichkeit und somit verzögerte Wirkstofffreisetzung, der hohe Anteil an unwirksamen jedoch für die Verarbeitung notwendigen Hilfs- und Füllstoffen, die in der Regel schlechte Eignung zur äußerlichen Anwendung sowie eine unzureichende Dosierbarkeit und damit sichere Handhabbarkeit bzw. Probleme bei der Applikation durch den Endanwender.

Eine geeignete und bekannte Möglichkeit, Wirkstoffe in einer gut löslichen trockenen Applikations- oder Dosierform bereitzustellen besteht darin, die Wirkstoffe in einem System von Trägerstoffen zu dispergieren und diese Mischung der Gefriertrocknung zu unterziehen. Als Trägerstoffe werden hierbei zumeist solche Substanzen gewählt, die ein gutes Auflösungs- bzw. Quellverhalten aufweisen und dabei durch die Quellung eine gute Texturbildung ermöglichen, so dass das aufgelöste bzw. in dem Quellstoff dispergierte Wirkstoffsystem direkt als Applikationsform einsetzbar ist. Solche Systeme sind aufgrund der guten Löslichkeit bekannt und geeignet für die Bereitstellung oraler Applikationsformen als auch für die Herstellung kosmetischer oder pharmazeutischer Darreichungsformen für die äußerliche Anwendung. Hierbei ist verstärkt ein Interesse in der Bereitstellung von sogenannten single-unit Anwendungsformen vorhanden, was eine einfache und zielgenaue Wirkstoff-Dosis-Applikation für den Endanwender ermöglicht. Als single-unit Anwendungsformen werden hier Applikationssysteme verstanden, die im Gegensatz zu Pulvern oder Granulaten pro Anwendungseinheit die gewünschte und notwendige Wirkstoffmenge in einer einzigen Applikationseinheit enthalten wie beispielsweise Tabletten oder Kapseln, ohne dabei jedoch die Nachteile der erschwerten Schluckbarkeit, der schlechten Löslichkeit oder der fehlenden Eignung für die äußerliche Applikation mit sich zu führen.

Sowohl für die kosmetische als auch für die pharmazeutische Anwendung von Einzelapplikations-Formkörpern, sind relativ große Formkörper einheitlicher Form und Größe bevorzugt, da diese im Gegensatz zu Pulvern oder kleinen Pellets und Granulaten durch den Endverbraucher einfacher zu handhaben sind, so dass das Bestreben dahin ausgerichtet ist, Formkörper einer solchen Größe bereitzustellen, die es erlauben pro Anwendung eine Dosierungsform zu benutzen. Außerdem vermitteln größere Formkörper, die z.B. farblich gestaltet werden können, auch einen stärkeren ästhetischen Eindruck.

Somit werden vermehrt größer formatige Ausgestaltungen solcher durch Trocknung stabilisierten single-unit Applikationsformen für die orale und/oder äußerliche Verabreichung von Wirkstoffen interessant, die sich durch ein besonders schnelles, zuverlässiges und vollständiges Löslichkeitsverhalten möglichst ohne inhomogene unvollständig gelöste Rückstände auszeichnen.

Das Auflösungsverhalten eines Formkörpers ist eine komplexe Funktion physikalisch/chemischer Parameter. Die physikalische Basis für den kinetischen Aspekt des Hydratationsprozesses bilden Einzelparameter wie Quellfähigkeit der verwendeten Trägerstoffe, strukturelle Integrität des Formkörpers unter Quellung, Porengröße, Kapillarkräfte verursacht durch die Porenstruktur, Oberflächenspannung verändernde Rezepturkomponenten, Dichte des Formkörpers etc.. Neben der reinen "Löslichkeit", oder im Falle von Trägerstoffen auf Basis von Polymeren der "Quellfähigkeit", spielt dabei die mechanische Stabilität des Formkörpers eine elementare Rolle. Quillt das polymere Material zu schnell, verliert der Formkörper zu schnell seine physikalische Integrität und kollabiert. Der Formkörper schmilzt während der Feuchtigkeitszugabe und zwar in den allermeisten Fällen unter Bildung noch nicht gequollener "innerer" Bereiche mit geringerem Flüssigkeitsanteil.

Sind die stabilisierenden Wechselwirkungen innerhalb des Materials zu groß, quillt das Material zwar unter Wasserzugabe, zerfällt aber anschließend nur unter mechanischer Belastung, wie beispielsweise durch mechanisches Verrühren, Verreiben, Zerdrücken oder Massieren. Ein Mittelweg, genügende mechanische Festigkeit zur Kompensation der Gewichtszunahme unter Quellung und anschließender, schneller Hydratation ist äußerst schwierig zu erreichen.

Bekannte schnell-lösliche Dosierungsformen z. B. zur oralen Applikation von Wirkstoffen stellt die Firma R. P. Scherer bzw. die Firma Cardinal Health unter der Bezeichnung Zydis® her, wobei dabei die flüssige Wirkstoffzusammensetzung in eine Blisterverpackung abgefüllt und in der Endverpackung gefriergetrocknet wird. Dieses Verfahren ist aufgrund der geringen mechanischen Stabilität der dadurch erhältlichen leichtlöslichen oralen Dosierungsformen notwendig. Die mangelnde mechanische Stabilität ist jedoch nachteilig insbesondere für die äußerliche Anwendung. Außerdem sind dadurch lose handhabbare Formkörper mit ausreichender mechanischer Stabilität zur Verwendung als single-unit Applikationsformen mit besonders schnellem und vollständigem Auflösungsverhalten naheliegenderweise nicht erhältlich. Darüber hinaus ist ein Verfahren der Gefriertrocknung in Blisterverpackungen technisch aufwendig und kostenintensiv und die dadurch erhältlichen Darreichungsformen in ihren Präsentations- und Kombinationsmöglichkeiten auf herkömmliche Blisterverpackungen beschränkt, was insbesondere für kosmetische Anwendungen unerwünscht ist.

Andere schnell-lösliche Zubereitungen, die ebenfalls aufgrund mangelnder mechanischer Stabilität in Blisterverpackungen gefriergetrocknet werden, sind Gegenstand der US 4,758,0598 und US 4,305,502.

Dagegen grenzt sich die DE 69727922 ab, indem sie orale schnell-lösliche Verabreichungsformen mit höherer Stabilität herstellt, die relativ hohe Anteile an Füllstoffen wie z. B. Mannitol sowie Bindemittel wie z. B. Alginat enthalten und die nicht durch teure und aufwändige Gefriertrocknungsverfahren erhältlich sind. Die dadurch erhältlichen oralen Dosierungsformen zeichnen sich durch eine Löslichkeit von < 15 Sekunden aus. Aus der DE 69727922 lässt sich jedoch nicht entnehmen, dass mit dem beschriebenen Verfahren Formkörper mit ausreichender mechanischer Stabilität erhältlich sind, die insbesondere auch für eine äußerliche Anwendung notwendig ist. Darüber hinaus wird hier die Problematik der schnellen und vollständigen Auflösung ohne mechanische Einwirkung und dabei ohne die Bildung inhomogener nicht gequollener innerer Bereiche nicht thematisiert. Da die in der DE 69727922 beschriebenen Dosierungsformen ausschließlich für die orale Applikation gedacht sind, ist ein solches spezielles Auflösungsverhalten auch nicht unbedingt relevant, da natürlicherweise die Zungen- und Gaumenbewegungen zwangsläufig eine mechanische Einwirkung darstellen, die gegebenenfalls ungleichmäßig gelöste Bereiche bei der weiteren Auflösung unterstützt. Weitere bekannte Verfahren zur Bereitstellung schnell-löslicher Wirkstoff-Applikationsformen, die als single-unit Dosierungsformen mit einer gewissen Größe verwendet werden können beschreiben die US 5,405,616 und die DE 4201179. Die darin verwendeten Trägermaterialien sind vorzugsweise solche auf Basis von Proteinen. Die Pellets werden hergestellt, indem Dispersionen aus den Protein-Gerüstbildnern und gegebenenfalls kosmetischen oder pharmazeutischen Wirkstoffen in tiefkalte inerte Flüssigkeiten, vorzugsweise flüssigen Stickstoff, eingetropft werden und die gefrorenen Pellets anschließend abgetrennt und gefriergetrocknet werden. Um unter diesen Bedingungen Pellets zu bilden, ist jedoch die Anwesenheit von Protein-Gerüstbildnern, insbesondere Kollagen bzw. Kollagen-Derivaten erforderlich, da nur die genannten Protein-Gerüstbildner unter diesen Bedingungen stabile Pellets ausbilden können. Proteinogene Trägermaterialien sind jedoch zur Erreichung eines besonders schnellen und vollständigen Auflösungsverhaltens ohne Rückstände gequollener Bereiche und insbesondere ohne mechanische Einwirkung, wie hier gewünscht, aufgrund der hohen Anforderungen an eine intramolekulare Stabilisierung der Zusammensetzung im verwendeten Eintropfverfahren nicht geeignet.

Eine ähnliche Problematik ergibt sich aus dem in der US 5,843,347 dargestellten Verfahren zur Herstellung poröser galenischer Partikel, die laut beschreibendem Teil in Größen bis 5 mm erhalten werden sollen. Bei dem hierin verwendeten Verfahren wird eine Mischung der Wirkstoffe in einer Matrix extrudiert und in Partikel der gewünschten Größe geschnitten, die anschließend durch Gefriertrocknung die porösen Formkörper bilden. Die Ausführungsbeispiele, zeigen jedoch lediglich, dass sogenannte Mikroperlen mit Durchmessern bis maximal 1,5 mm erhältlich sind. Dies ist auf die erfindungsgemäße Verwendung eines Extrusions- und Schneidverfahrens zurückzuführen, welches eine gewisse mechanische Stabilität der extrudierten Masse voraussetzt. Dies kann in der Regel durch einen relativ hohen Anteil an gerüstbildenden Polymeren bzw. Trägerstoffen und Stabilisatoren oder Füllstoffen erreicht werden. Werden jedoch lediglich geringe Mengen stabilisierender Träger- oder Hilfsstoffe im Vergleich zum Wirkstoffgehalt eingesetzt, so können, wie in diesem Dokument gezeigt, sehr kleinformatige Mikroperlen erhalten werden.

Ein anderes Verfahren, das die Herstellung gut löslicher wirkstoffbeladener Formkörper bereitstellt ist Gegenstand der WO 05/073296 sowie der WO 04/011537, wobei beide Dokumente keine genaueren Angaben über die Art sowie insbesondere Geschwindigkeit der Auflösung machen. Speziell die besonders schnelle und rückstandsfreie Auflösung ohne mechanische Einwirkung wird nicht genannt.

Ein für die äußerliche Verabreichung von Wirkstoffen geeigneter schnell-löslicher großformatiger Formkörper ist Gegenstand der DE 10248314, auch als WO 2004/035023 veröffentlicht,. Die durch das dort beschriebene Verfahren erhältlichen Applikationsformen sind regelmäßig geformte großformatige mit Wirkstoffen beladene Formkörper, die erhältlich sind durch ein Gefriertrocknungsverfahren einer in Formen gegossenen Wirkstoff-Gerüstbildner-Mischung. Die nach dem beschriebenen Verfahren erhältlichen Formkörper zeichnen sich durch eine gute mechanische Stabilität und eine hohe Auflösungsgeschwindigkeit von < 4 Minuten aus, wobei diese allerdings prinzipiell abhängig vom jeweiligen Gehalt an Gerüstbildnern ist. Es werden Auflösungsgeschwindigkeiten bis < 20 Sekunden beschrieben, wobei eine derart schnelle Auflösung lediglich durch Anwendung mechanischer Kräfte z. B. in Form von Verrühren, Verreiben oder Massieren erreicht wird.

Um nun noch schneller und besser lösliche Dosierungsformen mit vollständiger und rückstandsfreier Zerfallswirkung erhalten zu können, die insbesondere ohne mechanische Einwirkung homogen und gleichmäßig zerfallen und dabei keine gequollenen inhomogenen Bereiche zurücklassen und die damit besonders geeignet sind zur Verabreichung von kosmetischen und pharmazeutischen Wirk-, Aktiv- und Pflegestoffen, sind somit aus dem Stand der Technik bisher keine geeigneten Verfahren und Zusammensetzungen bekannt.

Die Aufgabe der vorliegenden Erfindung bestand somit darin, eine Zusammensetzung zur Verabreichung von Wirkstoffen zur Verfügung zu stellen, die sich durch eine besonders schnelle Löslichkeit auszeichnet, wobei die Auflösung vollständig und möglichst rückstandsfrei ohne Bildung inhomogener gequollener Bereiche sowie insbesondere ohne mechanische Einwirkung erfolgen sollte und die ein gegenüber bekannten leicht-löslichen Darreichungsformen verbessertes Auflösungsverhalten aufweist. Darüber hinaus bestand die Aufgabe darin, diese besonders schnell- und vollständig löslichen Wirkstoff-Zusammensetzungen so auszugestalten, dass sie eine hohe mechanische Festigkeit und eine ausreichende Größe aufweisen, um insbesondere zur kosmetischen oder pharmazeutischen Applikation in Form von sogenannten singledosage-units oder Einzeldosisanwendung verwendet werden zu können. Dabei sollen die Zusammensetzungen gleichermaßen geeignet sein für die äußerliche Anwendung sowie für eine orale oder perorale Applikation.

Überraschend wurde gefunden, dass eine derartige schnelle und vollständige Auflösung erhalten werden kann, wenn das Auflösungsverhalten einen speziellen zweistufigen Ablauf erfährt, während dessen der Formkörper sich bei Kontakt mit dem Lösungsmittel bzw. dem Befeuchtungsmittel zuerst vollsaugt und dabei für einige Sekunden seine Form beibehält und anschließend unter Verlust seiner physikalischen Form in ein Gel zerfällt. Eine solche zweistufige Hydratationskinetik kann erhalten werden mit gefriergetrockneten Formkörpern, die eine Mischung mindestens zweier Rezepturkomponenten, Natrium-Alginat und Magnesiumascorbylphosphat enthalten. Zusätzliche Rezepturbestandteile wie kosmetische Öle wie z. B. Triglyceride, Jojobaöl oder Squalan, sowie kosmetische oder pharmazeutische Wirksubstanzen beeinflussen die prinzipielle Hydratationskinetik dabei nicht. Überraschender Weise können die Zusätze an Hilfsstoffen wie Öle dabei deutlich größer sein als die enthaltenen Mengen an Alginat oder Magnesiumascorbylphosphat. Die Öle sind zur Erreichung der speziellen Hydratationskinetik nicht zwingend erforderlich.

Es wird vermutet, dass das Magnesiumascorbylphosphat das Alginatgerüst in der Zusammensetzung derart stabilisiert, dass eine zusätzliche mechanische Kraft z. B. verursacht durch den hydrostatischen Druck bei der Quellung, für kurze Zeit aufgenommen werden kann. Erst nach vollständiger Hydratation ist das Stabilitätsgleichgewicht überschritten und der Formkörper zerfällt. Versuche mit der Substitution des Magnesiumascorbylphosphats durch diverse Magnesiumsalze (z.B. MgCl, MgSO₄ etc) oder auch Mischungen solcher Salze mit Ascorbinsäure oder durch weitere Ascorbinsäure-Derivate wie Ascorbylglucosid zeigen, dass dieser spezielle Hydratationseffekt nur über die Einarbeitung von Magnesiumascorbylphosphat erreicht wird.

Magnesiumascorbylphosphat ist aus dem Stand der Technik als Inhaltsstoff in kosmetischen Zubereitungen bekannt. So offenbart die JP 2231496 ein Verfahren zur Gefriertrocknung von Magnesiumascorbylphosphat für die kosmetische Anwendung, wobei ein gut lösliches Magnesiumascorbylphosphat Pulver erhalten wird.

Weitere Dokumente wie die US 6,458,379, DE 4002532 oder JP 2004-149468 beschreiben die Verwendung von Mägnesiumascorbylphosphat in kosmetischen Zubereitungen als Wirkstoff, insbesondere als hautaufhellenden sogenannten Whitening-Wirkstoff.

In keinem der genannten Dokumente des Stands der Technik werden gefriergetrocknete pharmazeutische oder kosmetische Formkörper mit einer derart speziellen Kombination von Alginat und Magnesiumascorbylphosphat beschrieben, die sich darüber hinaus durch ein derart spezielles Auflösungsverhalten auszeichnen.

Zwar offenbart die FR 2886845 feste Zubereitungen in Form von z. B. Kugeln zur Wirkstoffapplikation in der kosmetischen und pharmazeutischen Verwendung, die vor der Anwendung hydratisiert und aufgelöst werden, und die außerdem die spezielle erfindungsgemäße Kombination aus polymerem Alginat-Trägermaterial und Magnesiumascorbylphosphat aufweisen können, hierin liegt jedoch das Magnesiumascorbylphosphat in einer speziellen mit einem besonderen Maleinsäurecopolymer stabilisierten Form vor. Darüber hinaus finden sich in dem Dokument keine genaueren Angaben zu den tatsächlichen Zerfallseigenschaften und -geschwindigkeiten der beschriebenen Formkörper. Lediglich im Zusammenhang mit dem Ausführungsbeispiel 1 wird ein "sofortiges Auflösen" von gefriergetrockneten Zusammensetzungen angegeben, wobei die dort beschriebenen Zusammensetzungen kein Magnesiumascorbylphosphat sondern Ascorbinsäure enthalten. Mit Ascorbinsäure im Austausch gegen Magnesiumascorbylphosphat kann das besondere zweistufige Auflösungsverhalten jedoch nicht erzielt werden, wie in Vergleichsversuchen gezeigt wurde. Vielmehr ist die der FR 2886845 zugrunde liegende Aufgabe auch nicht in der Bereitstellung einer Darreichungsform mit modifiziertem Auflösungsverhalten, sondern in der Stabilisierung des Magnesiumascorbylphosphats zu sehen, welches hierin jedoch rein als Wirkstoff, nämlich als stabilisiertes Vitamin C oder Ascorbinsäure-Derivat, und nicht aufgrund seiner besonderen technischen Funktionen hinsichtlich des Hydratationsverhaltens der erhältlichen Formkörper verwendet wird. Hinweise auf die technischen Wirkungen von Magnesiumascorbylphosphat in Hinblick auf die Erzielung einer besonderen Hydratationskinetik können der FR 2886845 nicht entnommen werden. Auch macht das Dokument keine Angaben zu Zerfallszeiten insbesondere zu solchen, die < 30 Sekunden aufweisen oder zu einem speziell zu wählenden pH-Wert der Zubereitung von > 4.

Aus der WO 2008/020066 sind gefriergetrocknete Zusammensetzungen bekannt, die Natrium-Alginat sowie Wirkstoffe, die aus der Gruppe der Vitamine ausgewählt sein können, in stabilisierter Form, bevorzugt in Form von Wirkstoffderivaten und/oder -precursorn enthalten. Dabei wird als ein bevorzugtes Vitaminderivat Magnesiumascorbylphosphat genannt. Als konkrete Ausführungsformen sind jedoch nur solche offenbart, die Natrium-Alginat in Kombination mit Ascorbylglucosid enthalten. Auch ein Austausch von Magnesiumascorbylphosphat gegen Ascorbylglucosid führt nicht zu dem modifizierten besonderen zweistufigen Auflösungsverhalten, wobei sich der gefriergetrocknete Formkörper beim Anfeuchten erst unter Erhaltung seiner Form voll saugt und dann zerfällt, was ebenfalls in Vergleichsversuchen gezeigt wurde.

Die WO 2009/014347 offenbart kleinteilige Partikel mit einem Durchmesser um 15 µm, die durch Sprühtrocknung hergestellt werden und die als Antioxidantien Magnesiumascorbylphosphat sowie als Stabilisatoren z. B. Alginsäure enthalten können. Eine konkrete Ausführungsform, die Magnesiumascorbylphosphat und ein Alginat enthält wird nicht beschrieben. Auch werden keine Angaben zum Auflösungsverhalten der erfindungsgemäßen Partikel gemacht.

Die vorliegende Erfindung stellt somit gefriergetrocknete Formkörper bereit, die Magnesiumascorbylphosphat und Alginat sowie gegebenenfalls einen oder mehrere Wirkstoffe und/oder Hilfsstoffe enthalten und die sich aufgrund dieser spezifischen Zusammensetzung durch ein besonderes, modifiziertes Auflösungsverhalten auszeichnen. Die Erfindung stellt darüber hinaus auch ein Verfahren zur Herstellung dieser gefriergetrockneten Formkörper sowie deren Verwendungsmöglichkeit in der kosmetischen und pharmazeutischen Verwendung bereit.

Unter einem Formkörper im Sinne der Erfindung versteht man einen regelmäßig geformten geometrischen Körper, z.B. insbesondere Kugeln, Quader, Pyramiden, Sterne aber auch natürlichen Formen nachgebildete Formkörper wie z.B. solche in der Form von Tieren, wie z.B. Meerestieren, wie z.B. Seesterne, Meeresfrüchte, wie Muscheln, etc. Pflanzen und Pflanzenteilen, wie Blätter etc. Nach dem weiter unten beschriebenen Verfahren zur Herstellung der erfindungsgemäß verwendeten Formkörper sind alle diese Formen zugänglich. Erfindungsgemäß bevorzugt sind einheitliche regelmäßige sphärische Formen wie insbesondere eine Kugelgeometrie, da diese sich als besonders vorteilhaft aufgrund des besonders günstigen Oberfläche/Volumen Verhältnisses auszeichnet. Die Sublimationsstrecke durch das schon trockene Produkt ist nach allen Seiten hin symmetrisch und klein, was den Dampftransport durch das schon trockene Gut im Rahmen des Gefriertrocknungsprozesse erleichtert und somit den Gefriertrocknungsprozess verkürzt und damit effizienter und kostengünstiger gestaltet und außerdem die Stabilität der erhaltenen Formkörper positiv beeinflusst.

Erfindungsgemäß ist auch eine Mehrzahl der genannten Formkörper in einem Behältnis umfasst. Auch kann es sich um Mischungen von Formkörpern verschiedener Geometrien oder unterschiedlicher Größen handeln. Die Formkörper können einzeln abgepackt sein, bevorzugt liegt jedoch insbesondere in der kosmetischen Anwendung eine Mehrzahl der Formkörper nebeneinander in Kontakt in einem Behältnis vor.

Die Volumina der verwendeten Formkörper sind aufgrund des Verfahrens ihrer Herstellung an sich nicht beschränkt. Zweckmäßig liegen die Volumina bevorzugt bei mindestens etwa 0,1 cm³, bevorzugt 0,3 cm³, bevorzugter mindestens etwa 0,5 cm³, noch bevorzugter mindestens etwa 0,7 cm³. Nach oben werden die verwendeten Volumina zweckmäßig auf bis zu etwa 6 cm³, bevorzugt bis zu etwa 5 cm³, bevorzugter bis zu etwa 4 cm³ beschränkt. Die Größe der Formkörper wird unter anderem durch die gewünschte Applikationsform oder den Ort der äußeren Anwendung der Formkörper bestimmt. So ermöglicht bei der äußeren kosmetischen oder pharmazeutischen Verwendung die Applikation auf größeren Körperflächen oder den Haaren (z.B. der direkte Auftrag der angefeuchteten Formkörper auf dem Rücken etc., oder der Einsatz als Badezusatz) den Einsatz größerer Formkörper, wohingegen bei der Anwendung auf kleineren Körperpartien (z.B. Wange etc.) kleinere Formkörper bevorzugt sind.

Auch bei der Herstellung von Formkörpern zur oralen Applikation kann die Größe angepasst werden. So ist beispielsweise denkbar, die Formkörpergröße auf die entsprechende Zielgruppe abzustimmen, wobei denkbar ist, älteren Anwendern größere Formkörper mit besserer Handhabbarkeit und leichterem Handling anzubieten und z. B. jüngeren Anwendern oder Kindern solche, die in einem abgestimmten Verhältnis zu ihrer Körpergröße und der altersbedingt zu erwartenden Compliance bei der Anwendung stehen.

Der Durchmesser eines Formkörpers (maximaler Abstand zwischen zwei Punkten in einem Formkörper jedweder Geometrie) liegt zweckmäßig bei mindestens etwa 3 mm, bevorzugt mindestens etwa 5 mm, bevorzugter mindestens etwa 7 mm, noch bevorzugter mindestens etwa 8 mm bis hin zu zweckmäßig etwa 60 mm, bevorzugt etwa 50 mm, bevorzugter etwa 40 mm, noch bevorzugter etwa 30 mm.

Ein besonders bevorzugter Formkörper weist aus besagten Gründen eine im wesentlichen kugelförmige Geometrie auf, wobei der Durchmesser der Kugel zwischen 3 bis 30 mm, bevorzugt zwischen 5 und 20 mm, bevorzugter zwischen 7 und 15 mm, noch bevorzugter zwischen 8 und 13 mm liegt. Besonders bevorzugt sind Formkörper in Form einer Kugel mit einem Durchmesser von mindestens 6 mm.

Bei Magnesiumascorbylphosphat, das erfindungsgemäß in Kombination mit Alginat zur Erzielung der speziellen modifizierten, zweistufigen Hydratationskinetik bei der Herstellung der gefriergetrockneten Formkörper eingesetzt wird, handelt es sich um ein Derivat der Ascorbinsäure (Trivialname: Vitamin C, (R)-5-[(S)-1,2-Dihydroxyethyl]-3,4-dihydroxy-5H-furan-2-on), genauer um einen Phosphorsäure-Monoester der Ascorbinsäure. Magnesiumascorbylphosphat zeichnet sich gegenüber der Ascorbinsäure insbesondere durch eine höhere Stabilität gegenüber Licht, Temperatur, Feuchtigkeit und Oxidation aus. Allerdings weisen solche Esterderivate auch eine geringere biologische Verfügbarkeit als reine Ascorbinsäure auf. Dennoch wird Magnesiumascorbylphosphat als kosmetischer Wirkstoff weitläufig eingesetzt, insbesondere als stabilisiertes Vitamin C-Derivat, sowie besonders als Hautaufhellungs- oder -bleichungsmittel (sogenanntes Whitening). Dabei wird Magnesiumascorbylphosphat kosmetischen Zubereitungen in der Regel in Konzentrationen zwischen 3-5 % zugesetzt. Eine besondere technische Wirkung hinsichtlich der Stabilisierung von Gerüstbildnern wie beispielsweise Alginat oder der Einfluss auf die Hydratationseigenschaften von getrockneten Zubereitungen ist bisher nicht beschrieben.

Die erfindungsgemäßen Formkörper enthalten außerdem als Trägermaterial aus der Gruppe der hydrokolloid-bildenden Polysaccharide, d.h. (teilweise) wasserlöslichen, natürlichen Polymere, die in wässrigen Systemen Gele bzw. viskose Lösungen bilden, Alginat, insbesondere Natriumalginat.

Es können auch Mischungen aus Alginat und weiteren Gerüstbildnernern z. B. aus der Gruppe der Polysaccharide wie z. B. Carrageen, Pektine, Tragant, Guar-Gummi, Johannisbrotkernmehl, Agar-Agar, Gummi-Arabikum, Pullulan, Trehalose, Xanthan, natürliche und modifizierte Stärken wie z.B. kationisch modifizierte Stärken, Dextrane, Dextrin, Maltodextrine, Chitosan, Glucane, wie β-1,3-Glucan, ß-1,4-Glucan, wie Cellulose, aus der Gruppe der Mucopolysaccharide, wie Hyaluronsäure etc. oder den synthetischen Polymeren wie beispielsweise Celluloseether, Polyvinylalkohol, Polyvinylpyrrolidon, synthetische Cellulosederivate, wie Methylcellulose, Carboxycellulose, Carboxymethylcellulose, kationisch modifizierte Carboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc. verwendet werden.

Mit der vorliegenden erfindungsgemäßen Kombination von Alginat und Magnesiumascorbylphosphat konnten überraschender Weise solche Formkörper erhalten werden, die neben der besonderen mechanischen Stabilität bei dem Rehydratiationsprozess auch über eine ausgezeichnete Stabilität gegenüber mechanischem Abrieb z. B. bei Handling, Verpackung, Lagerung und Transport verfügen und es somit ermöglichen, nicht staubende Wirkstoff-Formkörper bereitstellen zu können.

Die erfindungsgemäß bevorzugt verwendeten Alginate und ggf. weiteren Gerüstbildner weisen zweckmäßig durchschnittliche Molmassen von etwa 10³ bis zu etwa 10⁸, bevorzugt etwa 10⁴ bis 10⁷ auf.

Die Alginate und ggf. weiteren Gerüstbildner sind haut- und schleimhautverträglich und weisen weder bei der äußerlichen noch bei der oralen oder peroralen Applikation toxikologisches Potential, Irritationswirkungen oder andere Unverträglichkeitsreaktionen hervor. Sie sind pharmakologisch völlig unbedenklich und somit optimal geeignet als Trägermaterial für die erfindungsgemäßen kosmetischen und pharmazeutischen äußerlichen und oralen bzw. peroralen Verwendungen.

Die erfindungsgemäßen gefriergetrockneten Formkörper können gegebenenfalls neben dem Alginat und dem Magnesiumascorbylphosphat zusätzlich auch mindestens einen oder mehrere Wirkstoffe enthalten. Dabei ist anzumerken, dass Magnesiumascorbylphosphat an sich ebenfalls eine kosmetische oder therapeutische Wirkung besitzt und außerdem als kosmetischer Wirkstoff weit verbreitet ist und häufig eingesetzt wird. In der vorliegenden Erfindung wird das Magnesiumascorbylphosphat jedoch primär aufgrund seiner besonderen technischen Funktion bei der Stabilisierung der Formkörper im Auflösungsprozess eingesetzt. Es ist somit nicht als Wirkstoff im Sinne der anspruchsgemäßen Erfindung zu verstehen.

Auch die Alginate und ggf. weiteren Gerüstbildner können gewisse therapeutische Wirkungen aufweisen. So wirkt der bevorzugt verwendete Gerüstbildner Natrium-Alginat in gewissem Ausmaß antiviral und Hyaluronsäure wird eine gewisse Wirkung in der Re-Epithelisierung und als Antioxidans und Feuchtigkeitsspender in der Hautpflege nachgesagt, auch sie sind jedoch nicht als Wirkstoffe im Sinne der Erfindung zu verstehen.

Wirkstoffe schließen insbesondere kosmetische oder therapeutische bzw. pharmazeutische, für die äußere Anwendung sowie für die orale oder perorale Applikation geeignete Wirkstoffe ein. Bevorzugt enthält der erfindungsgemäß verwendete Formkörper mindestens einen kosmetischen und/oder pharmazeutischen Wirkstoff. Dementsprechend handelt es sich bei den erfindungsgemäßen gefriergetrockneten Formkörpern bevorzugt um kosmetische oder therapeutische Mittel.

Kosmetische Formkörper bzw. unter Verwendung kosmetischer Wirkstoffe hergestellte Formkörper im Sinne der Erfindung sind im wesentlichen Mittel im Sinne des Lebens-mittel-, Bedarfsgegenstände- und Futtermittelgesetzbuches (LFGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielweise um Badepräparate, Hautwasch- und reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -aufhellungsmittel, Depigmentierungsmittel, Deodorants, Antihydrotika, Haarentfernungsmittel, Insektenrepellents etc. oder derartige Mittel in Kombination.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern, .Wirkstoffe mit reizender Nebenwirkung, wie alpha-Hydroxysäuren, ß-Hydroxysäuren, alpha-Ketosäuren, ß-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc., sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind natürliche und synthetische Feuchthaltefaktoren wie z. B. Glycerin, Harnstoff und Ceramide, Hautschutzmittel, Hautaufheller, Vitamine, Antioxidantien, sogenannte Antiagingmittel, antiirritative Mittel, Sonnenschutzmittel, etc.

Im Unterschied zu den vorstehend beschriebenen im wesentlichen in der Kosmetik verwendeten Formkörpern handelt es sich bei den therapeutisch verwendeten Formkörpern (Arzneimittel) um solche, die mindestens einen pharmazeutischen bzw. therapeutischen insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Solche Mittel bzw. Wirkstoffe sind sowohl für die äußere Anwendung als auch für die orale oder perorale Applikation bestimmt.

Bei Wirkstoffen für die äußere Anwendung handelt es sich insbesondere um hautaktive aber auch um transdermale Wirkstoffe. Sie schließen beispielsweise ein: Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und - derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, einschließlich antibakterielle Mittel wie kolloidales Silber und Silbersalze, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die äußerliche Anwendung.

Bevorzugte therapeutische Mittel für die äußere Anwendung sind Analgetika, z.B. Immunsuppressiva, Hormone, Mittel zur Behandlung von Hautkrankheiten, wie der Neurodermitis, der atopischen Dermatitis etc., und Anti-Herpesmittel.

Therapeutische Wirkstoffe für die orale oder perorale Applikation können ausgewählt sein aus der Gruppe der Antihistaminika, der Antibiotika, der Peptidarzneistoffe, Antimykotika, Bronchialtherapeutika wie Antiasthmatika, Antitussiva, Mucolytica etc., Antidiabetica wie z. B. Glibenclamid, Hormone, Steroidhormone wie Dexamethason, Herzglycoside wie Digitoxin, Herz- und Kreislauftherapeutika wie z. B. Beta-Blocker, Antiarrhythmika, Antihypertonika, Calcium-Antagonisten etc., Psychopharmaka und Antidepressiva wie z. B. trizyklische Antidepressiva (NSMRI), Serotonin-Wiederaufnahme-Hemmer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z. B. Antimigränemittel, Paracetamol, Salicylsäure und -derivate wie Acetylsalicylsäure, Diclophenac, Ibuprofen, Ketoprofen, Naproxen etc., Antiphlogistika, Vasodilatatoren, Diuretica, Gichtmittel, Zytostatika, Muskelrelaxantien, Pflanzenextrakte, Provitamine wie z. B. Beta-Carotin, Vitamine wie z. B. Vitamin C, A, B, E etc., Kieselerde, Mineralstoffe und Spurenelemente wie z. B. Kalium, Magnesium, Calcium, Selen, lod etc., Diätsupplemente und Nahrungsergänzungsmittel usw. alle für die orale oder perorale Applikation.

Ein besonders bevorzugter pharmazeutischer Wirkstoff, der sowohl für die äußerliche als auch die orale oder perorale Applikation verwendet wird ist die Salicylsäure- und ihre Derivate wie Acetylsalicylsäure (ASS).

Bevorzugt verwendete Wirkstoffe sind insbesondere wasserlösliche Wirkstoffe, die sich in der wässrigen Mischung aus Alginat und Magnesiumascorbylphosphat besonders gut lösen. Es ist jedoch auch möglich, fettlösliche Wirkstoffe einzuarbeiten. Diese können bevorzugt in den Hilfsstoffen aus der Gruppe der Fettsubstanzen und Öle gelöst oder dispergiert werden, bevor sie der wässrigen Alginat-Magnesiumascorbylphosphat-Mischung zugesetzt werden.

Die erfindungsgemäß verwendeten Formkörper enthalten weiterhin gegebenenfalls einen oder mehrere Hilfsstoffe. Hilfsstoffe schließen ein: Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Fettsäureester, Ester von Fettalkoholen wie Triglyceride und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, pH-Einstellungsmittel, wie Pufferstoffe, anorganische und organische Säuren oder Basen, oberflächenaktive Mittel neben den oben erwähnten Waschtensiden, wie Dispergiermittel, Emulgatoren etc., Füllstoffe, Stabilisatoren, Cosolventien, pharmazeutisch und kosmetisch gebräuchliche oder sonstige Farbstoffe und Pigmente, insbesondere solche, die primär zur farblichen Gestaltung der Formkörper eingesetzt werden und nicht zur Applikation und farblichen Gestaltung am menschlichen Körper, wie solche Pigmente und Farbstoffe wie die unter der Gruppe der Wirkstoffe aufgeführten dekorativen Farbstoffe, Konservierungsmittel, Weichmacher, Schmiermittel bzw. Gleitmittel, etc.

Ein besonders bevorzugter Hilfsstoff ist ausgewählt aus der Gruppe der pH-Einstellungsmittel, bevorzugt Natronlauge zur Einstellung des erfindungsgemäß gewünschten pH-Werts von ≥ 4, bevorzugt ≥ 4,5, bevorzugter ≥ 5,0, außerdem bevorzugter ≥ 5,5, noch bevorzugter ≥ 6,0 sowie ≥ 6,5.

Weist die Zusammensetzung aus Magnesiumascorbylphosphat und Alginat und gegebenenfalls weiteren Wirk- und Hilfsstoffen per se bereits den erfindungsgemäß gewünschten pH-Wert auf, so kann auf die Zugabe von pH-Einstellungsmitteln selbstverständlich verzichtet werden.

Weitere bevorzugte Hilfsstoffe sind solche, die ausgewählt sind aus der Gruppe der Fette und Öle, wie insbesondere aus der Gruppe der kosmetischen Öle. Dabei wird bevorzugt Squalan ausgewählt. Das Squalan bewirkt überraschend trotz seiner hydrophoben Beschaffenheit eine verbesserte Löslichkeit des Formkörpers, was die äußere Anwendung auf der Haut erleichtert. Squalan besitzt daneben auch hautpflegende Wirkungen, obwohl nicht Wirkstoff im Sinne der Erfindung. Weitere bevorzugte Hilfsstoffe aus der Gruppe der Fettsubstanzen und kosmetischen oder pharmazeutischen Öle sind das Neutralöl (Triglycerid) und Jojobaöl.

Die Einordnung der vorstehend erwähnten Stoffe in die Kategorie der Hilfsstoffe im Rahmen der vorliegenden Erfindung schließt nicht aus, dass diese Hilfsstoffe auch gewisse kosmetische und/oder therapeutische Wirkungen entfalten können, was maßgeblich für die genannten kosmetischen Öle gilt.

Wesentlich für die besondere zweistufige Zerfallskinetik, die maßgeblich für die besonders schnelle und vollständige, rückstandsfreie Auflösung der erfindungsgemäßen Formkörper ohne mechanische Einwirkung ist, ist der pH-Wert der Zusammensetzung. Dieser beträgt erfindungsgemäß ≥ 4,0, bevorzugt ≥ 5,0, bevorzugter ≥ 6,0, so dass eine 1 gewichtsprozentige Lösung oder Suspension der erfindungsgemäßen Formkörper in Wasser bei 20 °C somit einen pH Wert von ≥ 4,0, bevorzugt ≥ 4,5, bevorzugter ≥ 5,0, außerdem bevorzugter ≥ 5,5, noch bevorzugter ≥ 6,0 sowie ≥ 6,5 aufweist. Dabei hat sich überraschend gezeigt, dass je höher der pH-Wert ist, umso schneller die Auflösung der Formkörper erfolgt.

Die erfindungsgemäßen Formkörper dienen der äußeren kosmetischen und pharmazeutischen sowie der oralen oder peroralen Anwendung bei Menschen oder Tieren. Die äußere Anwendung erfolgt dabei so, dass der erfindungsgemäße Formkörper mit Wasser bzw. einer wässrigen Lösung, die einen oder mehrere Wirkstoffe und/oder einen oder mehrere Hilfsstoffe enthält, angefeuchtet oder gelöst wird. Bevorzugt werden dabei vorwiegend wässrige Lösungen verwendet, die außerdem hautbefeuchtende Alkohole wie Glycerin, Butylenglykol oder Pentylenglykol enthalten, sowie solche, die niedrigviskos sind (eine Viskosität < 50 mPas ,) und keine oder lediglich geringe Ölanteile (< 10 Gew.-% bezogen auf die Gesamtzusammensetzung der wässrigen Lösung) enthalten. Weiterhin bevorzugt sind solche Aktivatorlösungen, die frei sind von Erdalkaliionen wie insbesondere Calcium- und/oder Magnesiumionen (weniger als 1 Gew-% Calcium-und/oder Magnesiumionen bezogen auf die Gesamtzusammensetzung der wässrigen Lösung enthalten), sowie solche mit einem pH-Wert zwischen etwa pH 4,0 bis 8,0.

Bevorzugt wird der Formkörper von oben auf oder in die Oberfläche von 0,75 bis 5,0 ml der Flüssigkeit oder Aktivatorlösung gegeben. Die Flüssigkeitsmenge in der Applikation kann als Funktion der beabsichtigten Gelviskosität ausgesucht werden. Zweckmäßigerweise beträgt sie ca. das 10 bis 100fache des Kugelgewichtes. Für einen Formkörper mit einem Gewicht von ca. 50 mg sollte das Volumen der Hydratationslösung mindestens 0,75 ml betragen. Der Formkörper saugt sich dabei unter Erhalt seiner äußeren geometrischen Form mit der Aktivatorlösung voll, um dann innerhalb weniger Sekunden vollständig in eine homogene Lösung oder ein Gel zu zerfallen. Dieser Zerfallsprozess erfolgt ohne mechanische Einwirkung von außen beispielsweise durch Verrühren, Verreiben, Zerdrücken oder Massieren. Der Zerfall erfolgt dabei in ≤ 30 Sekunden, bevorzugt ≤ 20 Sekunden, bevorzugter ≤ 10 Sekunden. In einer besonders bevorzugten Ausführungsform läuft dieser spezielle Hydratations- und Auflösungsprozess in ≤ 5 Sekunden ab.

Erfindungsgemäß ist in der äußeren Anwendung ebenfalls enthalten, die Lösung des erfindungsgemäßen Formkörpers in einer für eine Badeanwendung geeigneten Wassermenge. Bevorzugt erfolgt die Anwendung jedoch so, dass die Formkörper mit einer kleinen Menge von etwa 0,5 bis 5,0 ml Wasser oder einer Wirk- und/oder Hilfsstofflösung, der sogenannten Aktivatorlösung, insbesondere mit einer Zusammensetzung wie vorstehend genannt, unter Bildung einer homogenen und einheitlichen rückstandsfreien Lösung oder eines Gels direkt auf der Haut oder im Haar oder in einem geeigneten Gefäß befeuchtet und aufgelöst und anschließend in gelöster Form auf die Haut oder auf das Haar aufgetragen wird.

Die vorliegende Erfindung betrifft auch eine Kombination, enthaltend mindestens einen der erfindungsgemäß verwendeten Formkörper sowie mindestens eine wässrige Aktivatorlösung, die einen oder mehrere Wirkstoffe und/oder mindestens einen oder mehrere Hilfsstoffe enthält, in einer zusammengehörenden, räumlichen Anordnung (Anwendungspaket, Set, Kit-of-Parts etc.). Bei der Wirkstofflösung kann es sich z.B. um Lösungen von leichtflüchtigen Wirk- und/oder Hilfsstoffen handeln, die aufgrund des Herstellverfahrens durch die Gefriertrocknung nicht in den Formkörper eingebracht werden sollen bzw. können, wie z.B. gewisse Anteile ätherischer Öle, Parfums etc. Auch können solche Wirk- und/oder Hilfsstoffe enthalten sein, die eine befeuchtende Wirkung erzielen, die insbesondere bei der äußerlichen Anwendung auf der Haut erwünscht und bevorzugt ist, und die aufgrund dieser befeuchtenden Wirkung oder aufgrund von Hygroskopie-Neigungen nicht in den erfindungsgemäßen gefriergetrockneten Formkörper eingearbeitet werden können, da dadurch dessen Stabilität beeinträchtigt werden kann. Des weiteren ist denkbar, der Aktivatorlösung solche Wirk- oder Hilfsstoffe zuzusetzen, die aus dem Magnesiumascorbylphosphat nach der Rehydratation durch chemische Umsetzung oder andere aktivierende Einflüsse (z. B. pH-Verschiebung o.a.) in der gebildeten Lösung oder dem Gel die weitaus wirksamere Ascorbinsäure (Vitamin C) freisetzen, so dass dem Magnesiumascorbylphosphat neben der stabilisierenden, die Hydratationskinetik beeinflussenden Wirkung außerdem eine Wirkstoff-Precursor-Funktion zukommt.

Die Ausgestaltung solcher Kit-of-parts Kombinationen aus erfindungsgemäßem Formkörper einerseits und Wirkstofflösung andererseits kann dabei vorsehen, dass die beiden Bestandteile separat aus der Kit-of-parts Anordnung entnommen werden und außerhalb davon für die weitere Verwendung zusammengeführt und aufgelöst werden. Es ist jedoch auch denkbar, dass eine Zusammenführung der beiden Komponenten innerhalb der Kit-of-parts-Verpackung selbst erfolgt und die aufgelöste Zusammensetzung sodann aus dieser direkt der weiteren kosmetischen oder pharmazeutischen äußerlichen, oralen und/oder peroralen Verwendung zugeführt wird. Dies kann bevorzugt direkt durch den Endverbraucher durchgeführt werden.

Die erfindungsgemäßen Formkörper enthalten bevorzugt ≥ 5 Gew.-%, bevorzugter ≥ 10 Gew.-%, noch bevorzugter ≥ 20 Gew.-%, besonders bevorzugt ≥ 25 Gew.% Magnesiumascorbylphosphat, bezogen auf das Gesamtgewicht des gefriergetrockneten Formkörpers.

Des weiteren enthalten die erfindungsgemäßen Formkörper bevorzugt ≥5 Gew.-%, bevorzugter ≥ 10 Gew.-%, noch bevorzugter ≥ 20 Gew.-%, besonders bevorzugt ≥ 25 Gew.% Alginat, insbesondere Natrium-Alginat, bezogen auf das Gesamtgewicht des gefriergetrockneten Formkörpers.

Das Mengenverhältnis von Magnesiumascorbylphosphat zu Alginat beträgt erfindungsgemäß bevorzugt 0,1:1 bis 5:1, bevorzugt 0,2:1 bis 3:1 bevorzugter 0,5:1 bis 2:1.

Die Formkörper können weiterhin bis zu 80 Gew.-% eines oder mehrerer Wirkstoffe, bevorzugter bis zu 40 Gew.-%, noch bevorzugter bis zu 25 Gew.-% eines oder mehrerer Wirkstoffe enthalten.

Besonders bevorzugt sind Wirkstoffe aus der Gruppe der Vitamine wie insbesondere Ascorbinsäure (Vitamin C) und ihre Derivate und/oder Salicylsäure und ihre Derivate wie Acetylsalicylsäure (ASS) und ihre Derivate.

Die Formkörper können außerdem bis zu etwa 80 Gew.-% eines oder mehrerer Hilfsstoffe bevorzugt bis zu 25 Gew.-%, bevorzugter bis zu 20 Gew.-% eines oder mehrerer Hilfsstoffe enthalten.

Bevorzugte Hilfsstoffe sind z.B. pH-Einstellungsmittel wie z. B. Natronlauge, kosmetische Öle, wie z. B. Squalan, Jojobaöl oder Neutralöl, oder Farbpigmente oder inerte Füllstoffe.

Die Formkörper enthalten gegebenenfalls auch Reste von Wasser. Da die gefriergetrockneten Formkörper aufgrund ihrer Inhaltsstoffe und ihres erfindungsgemäß sehr schnellen Auflösungsvermögens jedoch außerordentlich feuchtigkeitsempfindlich sind, ist der Wassergehalt so gering wie möglich zu halten. Der Wassergehalt kann dabei bei bis zu 10 Gew.-% liegen. Der Wassergehalt kann sich nach der Herstellung des Formkörpers durch Gefriertrocknung bei der Lagerung verändern, in der Regel erhöhen. Bevorzugt liegt der Wassergehalt des Formkörpers nach der Herstellung bei maximal 10 Gew.-%, bevorzugt bei weniger als 5 Gew.-%, bevorzugter bei weniger als 1 Gew.-%.

Ein besonders bevorzugter Formkörper enthält:
≥ 20 Gew.-% Magnesiumascorbylphosphat,
≥ 20 Gew.-% Alginat, bevorzugt Natriumalginate, besonders niedrig-viskoses calciumfreies Natriumalginat, dessen 1-gewichtsprozentige Lösung oder Suspension in Wasser (1 g in 99 ml Wasser bei 20°C, pH 6-8) bevorzugt eine Viskosität von weniger als 2000, bevorzugt weniger als 1000, besonders bevorzugt weniger als 100 mPas aufweist,
≤ 50 Gew.-% eines oder mehrerer Wirkstoffe,
bis zu 60 Gew.-% eines oder mehrerer Hilfsstoffe, wie insbesondere solche aus der Gruppe der kosmetischen Fette und Öle, wie insbesondere Triglyceride oder pH-Einstellungsmittel wie anorganische Basen, insbesondere z. B. Natronlauge, sowie -bis zu 10 Gew.-%, bevorzugt bis zu 5 Gew.-%, bevorzugter weniger als 1 Gew.-% Wasser,
mit der Maßgabe, dass der Formkörper sich bei Flüssigkeitszufuhr unter Erhalt seiner physikalischen Form voll saugt und anschließend ohne mechanische Einwirkung in ≤ 30, bevorzugt ≤ 20, bevorzugter ≤ 10, besonders bevorzugt ≤ 5 Sekunden vollständig zerfällt, und dass dessen 1 gewichtsprozentige Lösung oder Suspension in Wasser bei 20 °C einen pH Wert ≥ 4, bevorzugt ≥ 4,5, bevorzugter ≥ 5,5, noch bevorzugter ≥ 6,5 aufweist.

Bevorzugt weist der erfindungsgemäße Formkörper, wie z. B. der der vorstehend erwähnten Zusammensetzung, enthaltend Magnesiumascorbylphosphat und Alginat
- einen pH-Wert ≥ 4,5, bevorzugter ≥ 5,5, noch bevorzugter ≥ 6,5, gemessen in einer 1 gewichtsprozentigen Lösung oder Suspension des Formkörpers in Wasser bei 20 °C,
- eine Dichte von 0,005 g/cm³ bis zu 0,8 g/cm³ bevorzugt 0,01 g/cm³ bis zu 0,8 g/cm³,
- ein Volumen von 0,1 cm³ bis 6 cm³, bevorzugt 0,6 cm³ bis 6 cm³,
- einen Durchmesser (maximaler Abstand zwischen zwei Punkten des Formkörpers) von mindestens 6 mm auf und/oder
- besitzt bevorzugt eine sphärische Ausgestaltung, besonders bevorzugt die Form einer Kugel.

Die erfindungsgemäßen Formkörper stellen poröse Formkörper mit homogener Verteilung der Inhaltsstoffe im Sinne einer gleichen Verteilung der Inhaltsstoffe über den gesamten Formkörper dar.

Die erfindungsgemäßen Formkörper wie beispielsweise solche wie vorstehend genannt, werden bevorzugt mit einer wässrigen Flüssigkeit / Aktivatorlösung aufgelöst, die enthält:
- mindestens 70 Gew.-% Wasser,
- mindestens 5 Gew.-% mehrwertige Alkohole,
- bis zu 10 Gew.-% eines oder mehrerer Wirkstoffe wie insbesondere solche aus der Gruppe der kosmetischen Wirkstoffe
- bis zu 20 Gew.-% eines oder mehrerer Hilfsstoffe, wie insbesondere solche aus der Gruppe der kosmetischen Öle, wie insbesondere Capryl/Capronsäure-Triglyceride und die einen pH-Wert von 5 - 7 und des weiteren einen Gehalt an Erdalkaliionen wie insbesondere Calcium- und/oder Magnesiumionen von weniger als 1 Gew-% aufweist.

Die Auflösungsgeschwindigkeit der erfindungsgemäß verwendeten Formkörper beträgt ≤ 30 Sekunden, bevorzugt ≤ 20 Sekunden, bevorzugter ≤ 10 Sekunden, besonders bevorzugt ≤ 5 Sekunden, gemessen an Formkörpern mit 11 mm Durchmesser und einem Gewicht von 50 mg, gemessen durch Platzieren des Formkörpers auf eine Wasseroberfläche. Das Gewichtsverhältnis von Wasser zu Formkörper beträgt dabei ca. 5:1 bis 100:1. Solche erfindungsgemäßen Formkörper zeigen bereits nach < 5 Sekunden komplette Hydratation ohne erkennbaren nicht hydratisierten Kern.

Die erfindungsgemäßen Formkörper sind erhältlich durch ein Verfahren, das die folgenden Schritte umfasst:
(a) Herstellen einer wässrigen Suspension oder einer Lösung von Magnesiumascorbylphosphat und Alginat, sowie gegebenenfalls eines oder mehrerer Wirkstoffe und/oder Hilfsstoffe
(b)gegebenenfalls Einstellen des pH Werts der wässrigen Suspension oder Lösung auf pH > 4, bevorzugt > 4,5, bevorzugter > 5,5, besonders bevorzugt > 6,5
(c) Giessen der Mischung in eine Form
(d) Gefrieren der Mischung in der Form und
(e)Gefriertrocknung der gefrorenen Mischung unter Bildung des Formkörpers.

Gegebenenfalls können zwischen diesen Schritten weitere Schritte durchgeführt werden, insbesondere ist es möglich, nach dem Schritt (d) die gefrorenen Formkörper aus der Form zu entnehmen. Weiterhin ist möglich, daraufhin eine Bearbeitung der Oberfläche der gefrorenen Formkörper durch mechanische Bearbeitung durchzuführen.

Zweckmäßig geht man bei der Herstellung so vor, dass man zunächst eine wässrige Lösung des Alginats herstellt und anschließend das Magnesiumascorbylphosphat darin dispergiert und auflöst. Anschließend gibt man den oder die Wirkstoffe sowie gegebenenfalls einen oder mehrere Hilfsstoffe hinzu und vermischt. Gegebenenfalls wird nach Vermischen aller Bestandteile gegebenenfalls durch Zugabe der Hilfsstoffe aus der Gruppe der pH-Einstellungsmittel der gewünschte pH-Wert auf ≥ 4, bevorzugt ≥ 4,5, bevorzugter ≥ 5,0, außerdem bevorzugt ≥ 5,5, besonders bevorzugt ≥6,0 sowie ≥ 6,5 eingestellt. Bevorzugt erfolgt die Einstellung des pH-Wertes durch den Zusatz von anorganischen Basen, vorzugsweise durch den Zusatz von Natronlauge.

Die Menge der in der Lösung bzw. Suspension enthaltenen Feststoffe wie Trägermaterial Alginat und Magnesiumascorbylphosphat, sowie gegebenenfalls weitere Wirk- und Hilfsstoffe beeinflusst maßgeblich die Dichte (Gewicht des Formkörpers bezogen auf das Volumen der geometrischen Form des Formkörpers) des erhaltenen Formkörpers. Die Dichte ist wiederum eine wichtige Größe für die Porosität des Formkörpers und damit wiederum für die Auflösungsgeschwindigkeit des Formkörpers beim Befeuchten mit Wasser oder einer Wirk- und/oder Hilfsstofflösung. Die poröse Struktur gefriergetrockneter Formkörper ist eine wesentliche Grundlage für die schnelle Löslichkeit, da durch die große Oberfläche im porösen Material ein inniger Austausch zwischen wässriger Phase und festem Formkörper während des Rehydratisierungsprozesses stattfinden kann. Je höher die Konzentration der Wirkstoffe, des Gerüstbildners sowie ggf. der Hilfsstoffe in der Lösung ist, umso höher wird die Dichte und damit umso geringer wird der Porositätsgrad des Formkörpers und umgekehrt. Allerdings hängt der Porositätsgrad der Formkörper nicht allein von der Materialdichte ab. Vielmehr ist die Materialporosität im wesentlichen eine Funktion zweier Parameter, der Materialdichte und der Eiskristallgröße. Hohe Feststoffgehalte in der wässrigen Suspension erhöhen die Materialdichte im gefriergetrockneten Endprodukt und verringern die Kontaktfläche Rehydratisierungsmittel/Feststoff. Hohe Einfriergradienten führen zu kleinen Eiskristallen, welche zu großen inneren Materialoberflächen führen, was wiederum die Rehydratisierung begünstigt. Zur schnellen Befeuchtung und Auflösung der gefriergetrockneten Formkörper sind also niedrige Materialdichten und kleine Eiskristalle vorteilhaft, wobei zur Erzielung des für die spezielle zweistufige Auflösungskinetik der vorliegenden Erfindung andererseits auch ein gewisser Grad an Stabilität notwendig ist.

So spielt neben der reinen "Löslichkeit", oder im Falle von Trägerstoffen auf Basis von Polymeren wie Alginat der "Quellfähigkeit", auch die mechanische Stabilität des Formkörpers eine elementare Rolle. Quillt das Material zu schnell, verliert der Formkörper zu schnell seine physikalische Integrität und kollabiert, wobei der Formkörper sozusagen während der Feuchtigkeitszugabe schmilzt und zwar in den allermeisten Fällen unter Bildung noch nicht gequollener "innerer" Bereiche mit geringerem Flüssigkeitsanteil. Die so erhältliche Lösung oder das Gel ist inhomogen und weist eine ungleichmäßige Zusammensetzung und damit einhergehend auch eine ungleichmäßige Verteilung der Inhaltsstoffe auf.

Sind die stabilisierenden Wechselwirkungen innerhalb des Materials jedoch zu groß, quillt das Material zwar unter Wasserzugabe, zerfällt aber anschließend nur unter mechanischer Belastung, wie beispielsweise durch mechanisches Verrühren, Verreiben, Zerdrücken oder Massieren. Eine sorgfältige Abstimmung der Rezepturkomponenten und der Mengenverhältnisse in den angegebenen Bereichen ist somit erforderlich, um einerseits eine genügende mechanische Festigkeit zur Kompensation der Gewichtszunahme unter Quellung und andererseits einer anschließenden schnellen Hydratation zu erreichen.

Unter dem Gesichtspunkt Dichte/Porositätsgrad bzw. der Auflösungsgeschwindigkeit wird die Rezepturierung und Herstellung der erfindungsgemäßen Formkörper neben der Wahl des geeigneten Mengenverhältnisses an Magnesiumascorbylphosphat und Alginat außerdem so ausgerichtet, dass die Dichten der damit erhältlichen Formkörper zweckmäßig bei etwa 0,01 g/cm³ bis zu 0,8 g/cm³, bevorzugt etwa 0,015 g/cm³ bis zu 0,5 g/cm³ bevorzugt etwa 0,02 g/cm³ bis zu 0,2 g/cm³ liegen. Der Begriff der Dichte, wie er vorliegend verwendet wird, bezeichnet das Gewicht des Formkörpers bezogen auf das Volumen der äußeren geometrischen Form des Formkörpers.

Das Gewicht der einzelnen Formkörper hängt natürlich von deren Größe ab. Im allgemeinen liegt das Gewicht der einzelnen Formkörper bei etwa 10 bis 200 mg, bevorzugt 20 bis 100 mg. Beispielsweise weisen Kugeln von 11 mm Durchmesser ein Gewicht im Bereich von bevorzugt 20 bis 80 mg, bevorzugter 30 bis 60 mg auf. Für Kugeln anderer Durchmesser berechnen sich entsprechend der Volumenänderung die Vorzugsbereiche.

Die Auswahl einer sphärischen Ausgestaltung wie insbesondere einer Kugelgeometrie beeinflusst darüber hinaus insbesondere im Zusammenspiel mit einer Einfriergeometrie bei der der Formkörper bei mindestens < -20 °C gleichzeitig von allen Seiten eingefroren wird die Trocknung der Formkörper außerordentlich positiv. Durch das günstige Oberfläche/Volumen Verhältnis der Kugelform ist die Sublimation durch das Produkt nach allen Seiten hin symmetrisch und auf kleine Strecken aufgerichtet, wodurch wiederum der Dampftransport während der Sublimation erleichtert wird. Dieses Verfahren, Frieren innerhalb einer Form beispielsweise durch Anblasen mit kalter Luft, reduziert darüber hinaus den Anteil nicht ausfrierbaren Wassers auf ein Minimum, wodurch anschließend bei höheren Temperaturen getrocknet werden kann, was wiederum die Kosten für den Gefriertrocknungsprozess erheblich senkt.

Die Herstellung der Lösung, die der Gefriertrocknung unterworfen wird, erfolgt bevorzugt so, dass zunächst eine Lösung des Alginats hergestellt wird und anschließend das Magnesiumascorbylphosphat eingearbeitet wird. Daraufhin werden die gegebenenfalls vorhandenen weiteren Wirk- und/oder Hilfsstoffe eingearbeitet. Werden öllösliche Wirkstoffe verwendet, werden diese bevorzugt in gegebenenfalls als Hilfsstoffe verwendeten Ölen (insbesondere Squalan Jojobaöl und Triglyceriden) gelöst und anschließend der Alginat-Magnesiumascorbylphosphat-Mischung zugesetzt. Diese Herstellweise besitzt den Vorteil, dass sich kurzzeitig stabile Lösungen bzw. Suspensionen bilden. Es werden keine Emulgatoren oder oberflächenaktive Substanzen wie z. B. Tenside benötigt, und es findet während der Verarbeitung keine Phasentrennung der Lösung oder Suspension bei Verwendung öllöslicher bzw. öliger Hilfs- bzw. Wirkstoffe statt.

Bevorzugt werden jedoch wasserlösliche Wirkstoffe verwendet.

Die so hergestellte Lösung oder Suspension weist bevorzugt eine Zusammensetzung von
- 0,25 - 10 Gew.-% Magnesiumascorbylphosphat,
- 0,5 - 5 Gew.-% Alginat,
- 0 - 20 Gew.-% eines oder mehrerer Wirkstoffe und
- 0 - 25 Gew.-% eines oder mehrerer Hilfsstoffe, jeweils bezogen auf die Gesamtzusammensetzung der wässrigen Suspension oder Lösung, auf.

Die Lösung oder Suspension wird dann in Formen gegossen, die den Formkörpern entsprechende Hohlräume der gewünschten geometrischen Formen aufweisen. Die Form besteht bevorzugt aus Kautschuk, Silikon-Kautschuk, vulkanisiertem Kautschuk (Gummi) etc. Bevorzugt sind Gummiformen. Die Formmaterialien können gegebenenfalls beschichtet sein. Die Hohlräume der Formkörper, in die die Lösung hineingegossen wird, weisen im allgemeinen die Form des gewünschten Formkörpers auf. D.h., dass das Volumen des Hohlraums im wesentlichen dem Volumen der später erhaltenen Formkörper entspricht.

Da das Volumen der in die Hohlräume eingefüllten Lösungen bzw. Suspensionen beim Gefrieren zunimmt (Dichteunterschied zwischen Wasser und Eis), werden die Hohlräume in der Regel nicht vollständig gefüllt. Es werden auf diese Weise vollständig symmetrische Formkörper erhalten. Dies ist beispielsweise nach dem Verfahren durch Eintropfen in tiefkalte Lösungen (wie in flüssigen Stickstoff) nicht möglich, da es dort zu unsymmetrischen Temperaturverteilungen kommt, so dass sich stets mehr oder weniger starke Abweichungen von einer regelmäßigen Form ergeben. Solche unregelmäßig geformten Formkörper sind jedoch gerade im Bereich der kosmetischen Endprodukte nicht erwünscht. Das bedeutet in der Regel, dass diese nach dem Eintropfverfahren hergestellten Formkörper einer mechanischen Nachbearbeitung bedürfen, was nach dem Verfahren, wie es erfindungsgemäß verwendet wird, nicht erforderlich ist. Bei nach dem Eintropfverfahren hergestellten Formkörpern wird eine solche Nachbearbeitung mit zunehmendem Volumen des Formkörpers immer erforderlicher, da bei diesem Verfahren deutliche äußerliche Unregelmäßigkeiten auftreten, die insbesondere bei größeren Formkörpern stärker in Erscheinung treten.

Nach dem Einfüllen der Lösung in die Hohlräume der Form wird die Lösung bzw. Suspension gefroren. Das Abkühlen bzw. Gefrieren der Lösung kann an sich in beliebiger Weise wie beispielsweise durch Anblasen mit kalter Luft, Abkühlen durch Aufbringen auf eine mit Kühlsole durchflossene Platte oder auch das Eintauchen der Formen in flüssige Gase, wie z.B. das Eintauchen in flüssigen Stickstoff erfolgen. Die Abkühlgeschwindigkeit beeinflusst dabei die Größe der gebildeten Eiskristalle. Diese beeinflussen wiederum die Porengrößenverteilung des gebildeten Formkörpers. Werden wenige große Kristalle gebildet, so weist der Formkörper wenige große Poren auf. Werden viele kleine Kristalle gebildet, weist der Formkörper viele kleine Poren auf. Die Kristalle werden um so kleiner, je höher die Abkühlgeschwindigkeit der Lösung bzw. Suspension ist. Wie bereits ausgeführt wird eine Einfriergeometrie bevorzugt, bei der die Formkörper bei mindestens < -20 °C in der Form gleichzeitig von allen Seiten eingefroren werden.

Die Gefriertemperatur, die erforderlich ist, hängt unter anderem davon ab, wie stark die Gefrierpunktserniedrigung durch die in der Lösung enthaltenden Wirkstoffe bzw. Hilfsstoffe ist. Zweckmäßig liegt die Temperatur unterhalb des Gefrierpunktes von Wasser bis zur Temperatur von flüssigem Stickstoff (- 196°C). Bevorzugt ist die Gefriertemperatur etwa -10 bis -80°C, besonders bevorzugt -20 bis - 50 °C Nach dem Gefrieren der Lösung bzw. Suspension werden die Formkörper aus der Form genommen und gegebenenfalls einer Nachbearbeitung unterzogen. Die Nachbearbeitung kann mechanisch erfolgen, z.B. durch eine Oberflächenbehandlung (Schleifen, Aufrauhen etc.). Anschließend werden die Formkörper der Gefriertrocknung unterworfen.

Die Gefriertrocknung kann in an sich bekannter Weise nach allgemein bekannten Gefriertrocknungsverfahren, wie z.B. auch in der DE 4328329 C2, der DE 4028622 C2 oder der DE 10350654 A1 beschrieben, erfolgen.

Die Erfindung umfasst insbesondere die folgenden bevorzugten Ausführungsformen:
1. Gefriergetrockneter Formkörper enthaltend Magnesiumascorbylphosphat und Alginat sowie gegebenenfalls einen oder mehrere Wirkstoffe und/oder Hilfsstoffe.
2. Gefriergetrockneter Formkörper nach Ausführungsform 1 worin das Alginat Natrium-Alginat ist.
3. Gefriergetrockneter Formkörper nach einer der Ausführungsformen 1 bis 2 worin als Wirkstoff mindestens einer aus der Gruppe der Vitamine und deren Derivate, bevorzugt aus der Gruppe des Vitamin C und seiner Derivate ausgewählt ist.
4. Gefriergetrockneter Formkörper nach einer der Ausführungsformen 1 bis 3, worin als Hilfsstoffe mindestens einer aus der Gruppe der kosmetischen und/oder pharmazeutischen Öle ausgewählt ist.
5. Gefriergetrockneter Formkörper nach Ausführungsform 4 worin aus der Gruppe der Öle Triglyceride, Jojobaöl und/oder Squalan ausgewählt ist.
6. Verfahren zur Herstellung eines gefriergetrockneten Formkörpers gemäß einer der Ausführungsformen 1 bis 5 das die Schritte umfasst
   a. Herstellen einer wässrigen Suspension oder einer Lösung von Magnesiumascorbylphosphat und Alginat, sowie gegebenenfalls eines oder mehrerer Wirkstoffe und/oder Hilfsstoffe
   b. gegebenenfalls Einstellen des pH Werts der wässrigen Suspension oder Lösung auf pH > 4
   c. Giessen der Mischung in eine Form
   d. Gefrieren der Mischung in der Form und
   e. Gefriertrocknung der gefrorenen Mischung unter Bildung des Formkörpers.
7. Verfahren nach Ausführungsform 6, worin die wässrige Suspension oder Lösung aus Schritt (a)
   0,25 - 10 Gew.-% Magnesiumascorbylphosphat,
   0,5 - 5 Gew.-% Alginat, vorzugsweise Natrium-Alginat,
   0 - 20 Gew.-% eines oder mehrerer Wirkstoffe und
   0 - 25 Gew.-% eines oder mehrerer Hilfsstoffe enthält, jeweils bezogen auf die Gesamtzusammensetzung der wässrigen Suspension oder Lösung.
8. Verfahren nach einer der Ausführungsformen 6 bis 7 , worin das Einstellen des pH-Werts in Schritt (b) durch den Zusatz von anorganischen Basen, vorzugsweise durch den Zusatz von Natronlauge erfolgt.
9. Verfahren nach einer der Ausführungsformen 6 bis 8, worin die gefrorene Mischung aus Schritt (d) vor der Gefriertrocknung gemäß Schritt (e) aus der Form genommen wird.
10. Gefriergetrockneter Formkörper erhältlich nach dem Verfahren nach einer der Ausführungsformen 6 bis 9.
11. Gefriergetrockneter Formkörper nach einer der Ausführungsformen 1 bis 5 und 10 dessen 1 gewichtsprozentige Lösung oder Suspension in Wasser bei 20 °C einen pH Wert ≥ 4,0, bevorzugter ≥ 5,0, noch bevorzugter ≥ 6,0 aufweist.
12. Gefriergetrockneter Formkörper nach einer der Ausführungsformen 1 bis 5 und 10 bis 11, der bei Flüssigkeitszufuhr in ≤ 30 Sekunden, bevorzugt ≤ 20 Sekunden, bevorzugter ≤ 10 Sekunden, noch bevorzugter ≤ 5 Sekunden vollständig zerfällt.
13. Gefriergetrockneter Formkörper nach einer der Ausführungsformen 1 bis 5 und 10 bis 12, der durch eine zweistufige Hydratationskinetik gekennzeichnet ist, worin der Formkörper bei Kontakt mit einer Benetzungsflüssigkeit diese unter Erhalt seiner physikalischen Form aufnimmt und anschließend unter Verlust seiner physikalischen Form vollständig zerfällt.
14. Gefriergetrockneter Formkörper nach Ausführungsform 13, worin der Formkörper bei Kontakt mit einer Benetzungsflüssigkeit in einem Gewichtsverhältnis von Benetzungsflüssigkeit zu Formkörper von 5:1 bis 100:1 diese unter Erhalt seiner physikalischen Form aufnimmt und anschließend unter Verlust seiner physikalischen Form vollständig zerfällt.
15. Gefriergetrockneter Formkörper nach einer der Ausführungsformen 1 bis 5 und 10 bis 14, worin das Gewichtsverhältnis von Magnesiumascorbylphosphat zu Alginat 0,1:1 bis 5:1 beträgt.
16. Gefriergetrockneter Formkörper nach einer der Ausführungsformen 1 bis 5 und 10 bis 15, der ein Volumen von 0,1 cm³ bis 6 cm³, eine Dichte von 0,01 g/cm³ bis 0,8 g/ cm³ und/oder die geometrische Form einer Kugel mit einem Durchmesser von mindestens 6 mm aufweist.
17. Verwendung des gefriergetrockneten Formkörpers nach einer der Ausführungsformen 1 bis 5 oder 10 bis 16 als kosmetisches Mittel.
18. Verwendung des gefriergetrockneten Formkörpers nach einer der Ausführungsformen 1 bis 5 oder 10 bis 16 als pharmazeutisches Mittel.
19. Verwendung nach Ausführungsform 17 oder 18, worin die Anwendung äußerlich auf Haut oder Haar erfolgt.
20. Verwendung nach einer der Ausführungsformen 17 bis 19, worin der gefriergetrocknete Formkörper mit Wasser oder einer wässrigen Lösung eines oder mehrerer Wirkstoffe und/oder gegebenenfalls Hilfsstoffe angefeuchtet wird und ohne mechanische Einwirkung in ≤ 30 Sekunden, bevorzugt ≤ 20 Sekunden, bevorzugter ≤ 10 Sekunden, noch bevorzugter ≤ 5 Sekunden zerfällt und anschließend in gelöster Form auf die Haut oder auf das Haar aufgetragen wird.
21. Verwendung nach Ausführungsform 20, worin die Auflösung zweistufig erfolgt, indem sich
   a. der Formkörper mit der zugeführten Flüssigkeit voll saugt und dabei seine Form beibehält und
   b. anschließend unter Verlust seiner physikalischen Form zerfällt.
22. Verwendung des gefriergetrockneten Formkörpers nach einer der Ausführungsformen 1 bis 5 oder 10 bis 16 zur oralen oder peroralen Applikation von Wirkstoffen.
23. Kit-of-parts Kombination enthaltend mindestens einen gefriergetrockneten Formkörper gemäß einer der Ausführungsformen 1 bis 5 oder 10 bis 16 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.
24. Verwendung der Kit-of-parts Kombination nach Ausführungsform 23 als kosmetisches Mittel.
25. Verwendung der Kit-of-parts Kombination nach Ausführungsform 23 als therapeutisches Mittel.
26. Verwendung nach einer der Ausführungsformen 15 bis 22 und 24 bis 25, die direkt durch den Endverbraucher erfolgt.
27. Verwendung von Magnesiumascorbylphosphat zur Modifizierung des Auflösungsverhaltens von gefriergetrockneten Formkörpern auf Basis von Alginaten.

Die Erfindung wird durch die folgenden Beispiele näher veranschaulicht.

### BEISPIELE

### Beispiel 1

**Natriumalginat-Magnesiumascorbylphosphat-Kugel, Durchmesser 9 mm**

20 g Na-Alginat werden unter Rühren in 960 g Wasser gegeben und homogen gemischt. Anschließend werden unter Rühren 20 g Magnesiumascorbylphosphat hinzugegeben, dabei wird die Mischung, die einen pH-Wert von pH 6,0-8,5 aufweist, auf einer Temperatur von 15-25 °C gehalten. Die homogene (entgaste) Mischung wird in Formen gegossen, unter Anblasen von Luft ausgefroren, aus der Form genommen und anschließend in an sich bekannter Weise gefriergetrocknet.

Der gefriergetrocknete Formkörper enthält:
50 Gew.-% Na-Alginat
50 Gew.-% Magnesiumascorbylphosphat

### Beispiel 2

### Natriumalginat-Magnesiumascorbylphosphat-Kugel, Durchmesser 11 mm

30 g Na-Alginat werden unter Rühren in 955 g Wasser gegeben und homogen gemischt. Anschließend werden unter Rühren 15 g Magnesiumascorbylphosphat hinzugegeben, dabei wird die Mischung, die einen pH-Wert 6-8,5 aufweist, auf einer Temperatur von 15-25°C gehalten. Die homogene (entgaste) Mischung wird in Formen gegossen, unter Anblasen von Luft ausgefroren, aus der Form genommen und anschließend in an sich bekannter Weise gefriergetrocknet.

Der gefriergetrocknete Formkörper enthält:
66,6 Gew.-% Na-Alginat
33,3 Gew.-% Magnesiumascorbylphosphat

### Beispiel 3

### Natriumalginat-Magnesiumascorbylphosphat-Kugel, Durchmesser 13 mm

20 g Alginat werden unter Rühren in 920g Wasser gegeben und homogen gemischt. Anschließend werden unter Rühren 20 g Magnesiumascorbylphosphat hinzugegeben, dabei wird die Mischung, die einen pH-Wert von pH 6,0-8,5 aufweist, auf einer Temperatur von 15-25°C gehalten. Anschließend werden 40 g Capryl/Capronsäure-Triglyceride homogen eingemischt und die homogene (entgaste) Mischung daraufhin in Formen gegossen, unter Anblasen von Luft ausgefroren, aus der Form genommen und anschließend in an sich bekannter Weise gefriergetrocknet.

Der gefriergetrocknete Formkörper enthält:
25 Gew.-% Na-Alginat
25 Gew.-% Magnesiumascorbylphosphat
50 Gew.-% Triglyceride

### Beispiel 4

### Natriumalginat-Magnesiumascorbylphosphat-Kugel, Durchmesser 15 mm

20 g Na-Alginat werden unter Rühren in 910 g Wasser gegeben und homogen gemischt. Anschließend werden unter Rühren 20 g Magnesiumascorbylphosphat hinzugegeben, dabei wird die Mischung, die einen pH-Wert 6,0-8,5 aufweist, auf einer Temperatur von 15-25 °C gehalten. Anschließend werden 40 g Triglyceride und 10 g Wirkstoffe wie Grüner Tee Extrakt homogen eingemischt und die homogene (entgaste) Mischung daraufhin in Formen gegossen, unter Anblasen von Luft ausgefroren, aus der Form genommen und anschließend in an sich bekannter Weise gefriergetrocknet.

Der gefriergetrocknete Formkörper enthält:
22 Gew.-% Na-Alginat
22 Gew.-% Magnesiumascorbylphosphat
44 Gew.-% Triglyceride
11 Gew.-% Wirkstoffe (z. B. Grüner Tee - Extrakt)

### Vergleichsversuch 1:

### Auflösekinetik als Funktion der Rezeptur

Vorlage: jeweils 1 ml RO-Wasser (reverse osmosis Wasser), mit ca. 20 °C.
Kugeldurchmesser: 11 mm.
Probe 1: erfindungsgemäßer gefriergetrockneter Formkörper nach Beispiel 3.
Probe 2: Vergleichsprobe; gefriergetrockneter Formkörper, hergestellt analog zu Beispiel 3, worin Magnesiumascorbylphosphat gegen Ascorbinsäure (Vitamin C) ausgetauscht ist.

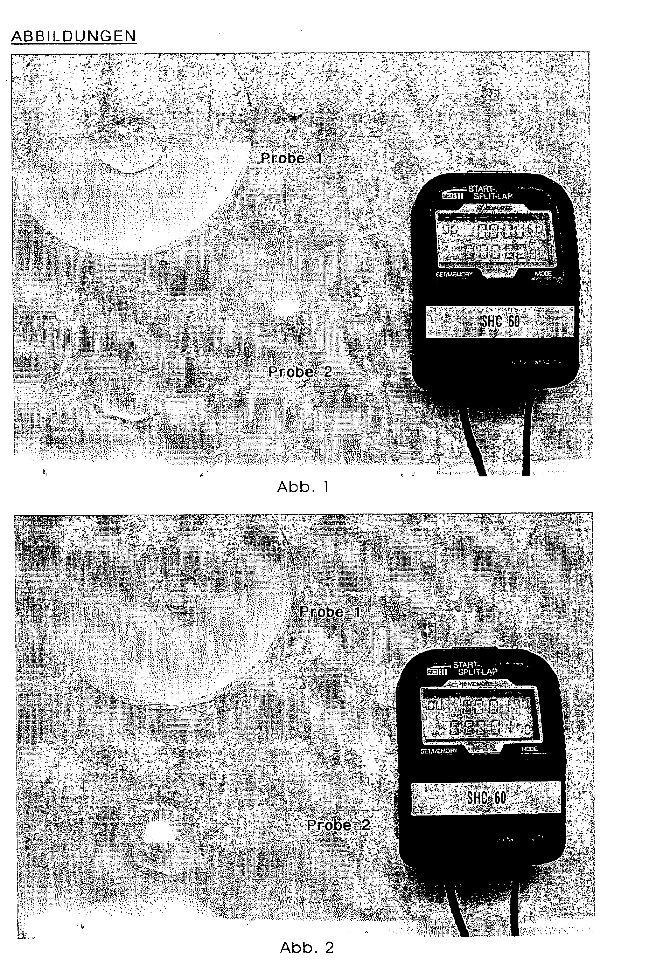

Abb. 1 zeigt den Aufbau des Vergleichsversuchs, die wässrige Vorlage wird auf Uhrgläsern vorgelegt.
Abb. 2 zeigt, wie sich innerhalb der 1. Sekunde Probe 1 (erfindungsgemäßer Formkörper) mit dem vorgelegten Wasser voll saugt.

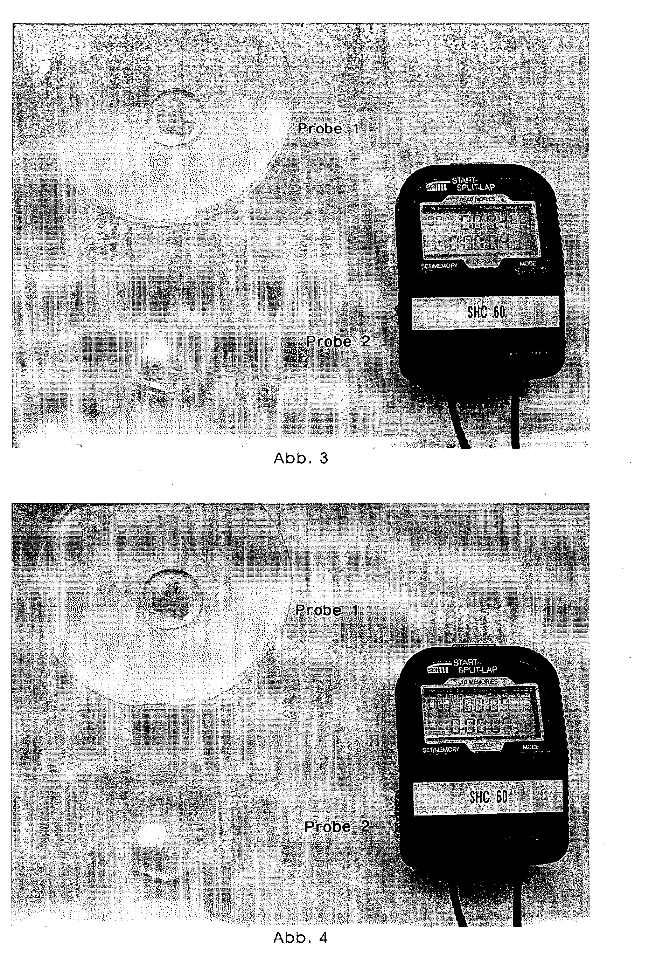

Abb. 3 zeigt weiter, dass Probe 1 nach ca. 4 Sekunden unter Verlust der strukturellen Integrität in ein vollständig hydratisiertes Gel ohne erkennbare nicht hydratisierte Bereiche zerfällt.

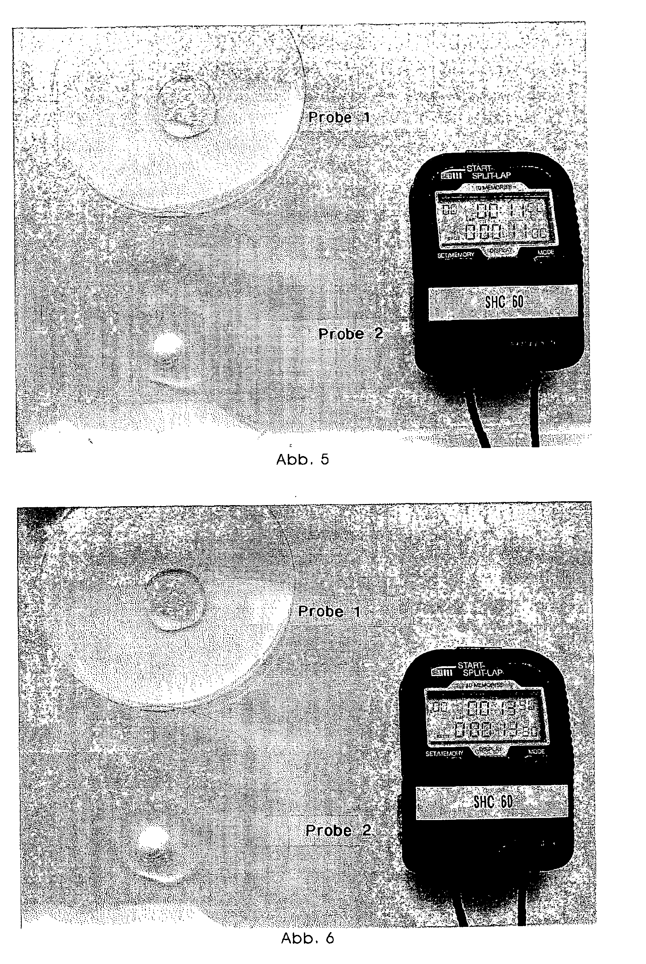

Die Abb. 4 bis 6 zeigen, dass sich die Vergleichsprobe 2 mit Vitamin C anstelle von MAP im weiteren zeitlichen Verlauf nicht vollständig voll saugt und auch nach 13 Sekunden (Abb. 6) noch einen oberen weißen, nicht hydratisierten Formkörperbereich aufweist.

Das hier dargestellte Auflöseverhalten ist prinzipiell unabhängig vom Kugeldurchmesser und grundsätzlich nicht beschränkt auf Formkörper mit einem Durchmesser von 11 mm, die nach Beispiel 3 hergestellt sind, sondern kann in gleicher Weise für alle Formkörper im Sinne dieser Erfindung mit unterschiedlicher Zusammensetzung und unterschiedlichen Durchmessern bzw. geometrischen Ausgestaltungen beobachtet werden.

### Vergleichsversuch 2:

Analog zu den Versuchsbedingungen nach Vergleichsversuch 1 wurde außerdem ein Vergleich der Auflösungsgeschwindigkeit und des Auflösungsverhaltens einer erfindungsgemäßen Zusammensetzung gemäß Beispiel 3 mit dem Vergleichsproben in Form eines gefriergetrockneten Formkörpers, hergestellt analog zu Beispiel 3, worin Magnesiumascorbylphosphat gegen Ascorbinsäure (Vitamin C) bzw. gegen Ascorbylglucosid ausgetauscht ist, durchgeführt. Das Ergebnis ist in Tabelle 1 dargestellt. Danach zeigt sich, dass der Ersatz von MAP gegen andere Vitamin C Verbindungen nicht das besonders schnelle und durch die zweistufige Kinetik gekennzeichnete Auflösungsverhalten zeigt. Vielmehr zeigte sich, dass sowohl die Vitamin C-Probe als auch die Ascorbylglucosid-Probe ein deutlich schlechteres Auflösungsverhalten zeigten. Ein Zerfall des Formkörpers war nicht ohne mechanische Einwirkung zu erzielen, somit konnte auch keine zweistufige Zerfallskinetik beobachtet werden.

**Tabelle 1**

| **Probe** | **Zweistufige Kinetik** | **Formerhalt vor Zerfall [s]** | **Auflösungs-/Zerfallsgeschwindigkeit [s]** | **Vollständige Auflösung / Zerfall** | **Mechanische Einwirkung notwendig** |
|---|---|---|---|---|---|
| **MAP** | + | 1.50 | 1 | + | - |
| **Vitamin C** | - | - | > 11 | - | + |
| **Ascorbylglucosid** | - | - | > 16 | - | + |

## Patentansprüche

1. Gefriergetrockneter Formkörper enthaltend Magnesiumascorbylphosphat und Alginat sowie gegebenenfalls einen oder mehrere Wirkstoffe und/oder Hilfsstoffe.

2. Gefriergetrockneter Formkörper nach Anspruch 1 worin das Alginat Natrium-Alginat ist.

3. Gefriergetrockneter Formkörper nach einem der Ansprüche 1 bis 2 worin als Wirkstoff mindestens einer aus der Gruppe der Vitamine und deren Derivate die aus der Gruppe die Glycoside, Ester und deren Alkalimetall- Erdalkalimetall- und trivalenten Metallsalze ausgewählt sind, bevorzugt aus der Gruppe des Vitamin C (Ascorbinsäure) und seiner Derivate ausgewählt ist.

4. Gefriergetrockneter Formkörper nach einem der Ansprüche 1 bis 3, worin als Hilfsstoffe mindestens einer aus der Gruppe der kosmetischen und/oder pharmazeutischen Öle ausgewählt ist.

5. Verfahren zur Herstellung eines gefriergetrockneten Formkörpers gemäß einem der Ansprüche 1 bis 4 das die Schritte umfasst
(a)Herstellen einer wässrigen Suspension oder einer Lösung von Magnesiumascorbylphosphat und Alginat, sowie gegebenenfalls eines oder mehrerer Wirkstoffe und/oder Hilfsstoffe
(b)gegebenenfalls Einstellen des pH Werts der wässrigen Suspension oder Lösung auf pH > 4
(c)Giessen der Mischung in eine Form
(d)Gefrieren der Mischung in der Form und
(e)Gefriertrocknung der gefrorenen Mischung unter Bildung des Formkörpers.

6. Verfahren nach Anspruch 5, worin die wässrige Suspension oder Lösung aus Schritt (a)
0,25 - 10 Gew.-% Magnesiumascorbylphosphat,
0,5 - 5 Gew.-% Alginat,
0 - 20 Gew.-% eines oder mehrerer Wirkstoffe und
0 - 25 Gew.-% eines oder mehrerer Hilfsstoffe enthält, jeweils bezogen auf die Gesamtzusammensetzung der wässrigen Suspension oder Lösung.

7. Gefriergetrockneter Formkörper nach einem der Ansprüche 1 bis 4 oder erhältlich nach dem Verfahren nach einem der Ansprüche 5 bis 6, dessen 1 gewichtsprozentige Lösung oder Suspension in Wasser bei 20 °C einen pH Wert ≥ 4,0, bevorzugter ≥ 5,0, noch bevorzugter ≥ 6,0 aufweist.

8. Gefriergetrockneter Formkörper nach einem der Ansprüche 1 bis 4, 7 oder erhältlich nach dem Verfahren nach einem der Ansprüche 5 bis 6, der bei Flüssigkeitszufuhr in ≤ 30 Sekunden, bevorzugt ≤ 20 Sekunden, bevorzugter ≤ 10 Sekunden, noch bevorzugter ≤ 5 Sekunden vollständig zerfällt.

9. Gefriergetrockneter Formkörper nach einem der Ansprüche 1 bis 4, 7 bis 8 oder erhältlich nach dem Verfahren nach einem der Ansprüche 5 bis 6, worin das Gewichtsverhältnis von Magnesiumascorbylphosphat zu Alginat 0,1:1 bis 5:1 beträgt.

10. Gefriergetrockneter Formkörper nach einem der Ansprüche 1 bis 4, 7 bis 9 oder erhältlich nach dem Verfahren nach einem der Ansprüche 5 bis 6zur Verwendung als pharmazeutisches Mittel.

11. Gefriergetrockneter Formkörper nach einem der Ansprüche 1 bis 4, 7 bis 10 oder erhältlich nach einem Verfahren nach einem der Ansprüche 5 oder 6 zur Verwendung zur oralen oder peroralen Applikation von Wirkstoffen.

12. Verwendung des gefriergetrockneten Formkörpers nach einem der Ansprüche 1 bis 4, 7 bis 9 oder erhältlich nach einem Verfahren nach einem der Ansprüche 5 oder 6 als kosmetisches Mittel.

13. Verwendung nach Anspruch 12, worin der gefriergetrocknete Formkörper mit Wasser oder einer wässrigen Lösung eines oder mehrerer Wirkstoffe und/oder gegebenenfalls Hilfsstoffe angefeuchtet wird und ohne mechanische Einwirkung in ≤ 30 Sekunden, bevorzugt ≤ 20 Sekunden, bevorzugter s 10 Sekunden, noch bevorzugter ≤ 5 Sekunden zerfällt und anschließend in gelöster Form auf die Haut oder auf das Haar aufgetragen wird.

14. Verwendung nach Anspruch 13, worin die Auflösung zweistufig erfolgt, indem sich
(a)der Formkörper mit der zugeführten Flüssigkeit voll saugt und dabei seine Form beibehält und
(b)anschließend unter Verlust seiner physikalischen Form zerfällt.

15. Kit-of-parts Kombination enthaltend mindestens einen gefriergetrockneten Formkörper gemäß einem der Ansprüche 1 bis 4, 7 bis 11 oder erhältlich nach einem Verfahren nach einem der Ansprüche 5 oder 6 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

## Claims

1. Freeze-dried molded article containing magnesium ascorbyl phosphate and alginate as well as optionally one or more active substances and/or auxiliary substances.

2. Freeze-dried molded article according to claim 1, wherein the alginate is sodium alginate.

3. Freeze-dried molded article according to any one of the claims 1 to 2, wherein, as an active substance, at least one is selected from the group of vitamins and their derivatives, which are selected from the group of glycosides, esters and their alkali metal-, alkaline earth metal- and trivalent metal salts, preferably from the group of vitamin C (ascorbic and its derivatives.

4. Freeze-dried molded article according to any one of the claims 1 to 3, wherein, as auxiliary substances, at least one is selected from the group of cosmetic and/or pharmaceutical oils.

5. Method for producing a freeze-dried molded article according to any one of the claims 1 to 4, comprising the following steps
(a) preparing an aqueous suspension or a solution of magnesium ascorbyl phosphate and alginate, as well as, optionally, one or more active substances and/or auxiliary substances
(b) optionally adjusting the pH- value of the aqueous solution or suspension to pH > 4,
(c) pouring the mixture into a mold
(d) freezing the mixture in the the mold, and
(e) freeze-drying the frozen mixture while forming the molded article.

6. Method according to claim 5, wherein the aqueous suspension or solution from step (a) contains
0.25 - 10 % by wt. magnesium ascorbyl phosphate,
0.5 - 5 % by wt. alginate,
0 - 20 % by wt. of one or more active substances and
0 - 25 % by wt. of one or more auxiliary substances, in each case based on the total composition of the aqueous suspension or solution.

7. Freeze-dried molded article according to any one of the claim to 4 or obtainable in accordance with the method according to any of the claims 5 to 6, whose 1 % by wt. solution or suspension in water, at 20°C, has a pH value ≥ 4.0, more preferably ≥ 5.0, still more preferably ≥ 6.0.

8. Freeze-dried molded article according to any one of the claims 1 to 4,7 or obtainable in accordance the method according to any of the claims 5 to 6, which decomposes completely within ≤ ≤ 30 seconds, preferably ≤ 20 seconds, more preferably ≤ 10 seconds, still more preferably ≤ 5 seconds when liquid is is added.

9. Freeze-dried molded article according to any one of the claims 1 to 4,7 to 8 or obtainable in accordance with the method according to any of the claims 5 to 6, wherein the weight ratio of magnesium ascorbyl phosphate to alginate is 0.1:1 to 5:1.

10. Freeze-dried molded article according to any one of the claims 1 to 4, 7 to 9 or obtainable in accordance with the method according to any of the claims 5 to 6 for the use as a pharmaceutical agent.

11. Freeze-dried molded article according to any one of the claims 1 to 4, 7 to 10 or obtainable in accordance with the method according to any of the claims 5 to 6, for the oral or peroral application of active substances.

12. Use of the freeze-dried molded article according to any one of the claims 1 to 4, 7 to 9 or obtainable in accordance with the method according to any of the claims 5 to 6, as a cosmetic agent.

13. Use according to claim 12, wherein the freeze-dried molded article is is moistened with water or an aqueous solution of one or more active substances and/or, optionally, auxiliary substances, and decomposes without mechanical influence within ≥ 30 seconds, preferably ≤ 20 seconds, more preferably ≤ 10 seconds, still more preferably ≤ 5 seconds, and is then applied onto the skin or hair in the dissolved form.

14. Use according to claim 13, wherein the dissolution takes place in two stages, by
(a) the molded article being completely soaked with the supplied liquid, retaining its shape in the process, and
(b) then decomposing while losing its physical shape.

15. Kit-of-parts combination, comprising at least one freeze-dried molded article according to any one of the claims 1 to 4, 7 to 11 or obtainable in accordance wit the method according to any of the claims 5 to 6, as well as at least one aqueous solution comprising one or more active substances and/or, optionally, one or more auxiliary substances, in a combined spatial arrangement.

## Revendications

1. Article moulé lyophilisé contenant du phosphate d'ascorbyle de magnésium et de l'alginate ainsi que facultativement une ou plusieurs substances actives et/ou substances auxiliaires.

2. Article moulé lyophilisé selon la revendication 1, dans lequel l'alginate est de l'alginate de sodium,

3. Article moulé lyophilisé selon l'une des revendications 1 ou 2, dans lequel, en tant qu'ingrédient actif, au moins un est sélectionnée parmi le groupe des vitamines et ses dérivés qui sont sélectionnées parmi le groupe des glycosides, des esters et ses de métaux alcalins, de métaux alcalino-terreux et de métaux trivalents, de façon préférée parmi le groupe de vitamine C (l'acide ascorbique) et ses dérivés.

4. Article moulé lyophilisé selon l'une des revendications 1 à 3, dans lequel, en tant que substances auxiliaires, au moins une est sélectionné parmi le groupe des huiles cosmétiques et/ou pharmaceutiques,

5. Procédé de production d'un article moulé lyophilisé selon l'une des revendications 1 à 4, comprenant les étapes suivantes consistant
(a) à préparer une suspension aqueuse ou une solution du phosphate d'ascorbyle de magnésium et de l'alginate ainsi que facultativement une ou plusieurs substances actives et/ou substances auxiliaires
(b) facultativement à ajuster le pH de ladite solution ou suspension à pH > 4
(c) à verser le mélange dans un moule
(d) à congeler le mélange dans le moule, et
(e) à lyophiliser le mélange congelé en formant l'article moulé.

6. Procédé selon la revendication 5, dans lequel la suspension aqueuse ou la solution de l'étape (a) contient
0,25 - 10% en poids de phosphate d'ascorbyle de magnésium,
0,5 - 5% en poids d'alginate,
0 - 20% en poids d'une ou plusieurs substances actives et
0 - 25 en poids d'une ou plusieurs substances auxiliaires, dans chaque cas sur la base de la composition totale de la suspension aqueuse ou de la solution.

7. Article moulé lyophilisé selon une des revendications 1 à 4 ou susceptible d'être obtenu par le procédé selon l'une des revendications 5 ou 6, dont la solution ou la suspension dans l'eau à 1 % en poids présent une valeur de pH à 20 °C ≥ 4.0, de façon préférée ≥ 5,0, de façon encore préférée ≥ 6,0.

8. Article moulé lyophllisé selon l'une des revendications 1 à 4, 7 ou susceptible d'être obtenu par le procédé selon l'une des revendications 5 ou 6, qui décompose totalement en ≤ 30 secondes, de façon préférée en ≤ 20 secondes, de façon encore préférée en ≤ 10 secondes, de façon particulièrement préférée en ≤ 5 secondes par addition d'un liquide.

9. Article moulé lyophilisé selon l'une des revendications 1 à 4, 7 à 8 ou susceptible d'être obtenu par le procédé selon l'une des revendications 5 ou 6, dans lequel le rapport pondéral du phosphate d'ascorbyle de magnésium à l'alginate est 0,1 : 1 à 5 :1.

10. Article moulé lyophilisé selon l'une des revendications 1 à 4, 7 à 9 ou susceptible d'être obtenu par le procédé selon l'une des revendications 5 ou 6 pour l'utilisation comme agent pharmaceutique.

11. Article moulé lyophilisé selon l'une des revendications 1 à 4, 7 à 10 ou susceptible d'être obtenu par le procédé selon l'une des revendications 5 ou 6 pour l'utilisation pour application orale ou préorale des substances actives.

12. Utilisation de l'article moulé lyophilisé selon l'une des revendications 1 à 4, 7 à 9 ou susceptible d'être obtenu par le procédé selon l'une des revendications 5 ou 6 comme agent cosmétique.

13. Utilisation selon la revendication 12, dans laquelle l'article moulé lyophilisé est humidifié avec de l'eau ou une solution aqueuse d'une ou plusieurs substances actives et/ou facultativement des substances auxiliaires et décompose sans influences mécaniques en ≤ 30 secondes, de façon préférée en ≤ 20 secondes, de façon encore préférée en ≤ 10 secondes, de façon particulièrement préférée en ≤ 5 secondes et est ensuite appliqué sur la peau ou les cheveux sous forme dissoute.

14. Utilisation selon la revendication 13, dans laquelle la dissolution se déroule en deux étapes, en
(a) complètement imbibant l'article moulé dans le liquide fourni, conservant sa forme dans le procédé et
(8) se décomposant ensuite en perdant sa forme physique.

15. Jeu de pièces constitutives à l'arrangement spatial combiné (« kit of parts ») comprenant au moins un article moulé lyophilisé selon l'une des revendication 1 à 4, 7 à 11 ou susceptible d'être obtenu par le procédé selon l'une des revendications 5 ou 6 et au moins une solution aqueuse comprenant une ou plusieurs substances actives et/ou facultativement une ou plusieurs substances auxiliaires.
